# EUROPEAN PATENT APPLICATION

(11) **EP 4 467 977 A1**
(43) Date of publication of application: **27.11.2024**
(21) Application number: 23020283.0
(22) Date of filing: 08.06.2023
(51) Int. Cl.: G01N 33/497, G01N 1/22, G01N 33/569

(54) **COLLECTION AND ANALYSIS OF ENVIRONMENTAL BIOLOGICAL MATERIAL**

(30) Priority: 26.05.2023 EP 23020262
(71) Applicant: SGS SOCIETE GENERALE DE SURVEILLANCE S.A., 1201 Geneve (CH)
(72) Inventor: The designation of the inventor has not yet been filed
(74) Representative: Roberts, Simon Christopher

(57) **Abstract**

Provided is a method of generating information about the biology within a survey zone, the method comprising:
i) harvesting biological material from an external impact surface that has been carried through the survey zone by a terrestrial vehicle, the surface having been exposed to air that is displaced by movement of the vehicle through the survey zone;
ii) performing molecular analysis on the biological material to generate data on sources of the biological material.

Also provided is a method of generating information on biological presence for a geographical area, the method comprising:
i) collecting biological material on an external impact surface of a terrestrial vehicle moved over the area;
ii) harvesting the collected biological material from the impact surface;
iii) performing molecular analysis on the harvested biological material to generate data on sources of the biological material.

Preferably the vehicle is a public transport vehicle such as a bus, tram, or train, or a road haulage or delivery vehicle.

## Description

### Technical field

The invention relates to improved sampling methods for collecting environmental biological material such as DNA and to methods of processing and using data obtained from such material.

### Background

It is now accepted that biodiversity is declining faster than at any previous time in human history. The International Union for Conservation of Nature (IUCN) reports that globally 42,100 species (almost a third of all assessed species) are threatened with extinction. The problem affects both the developed world and the developing world. It is reported that in the UK there has been on average a 70% decline in the populations of mammals, birds, fish, reptiles, and amphibians since 1970, while the UK's flying insect population has declined by as much as 60% in the last 20 years. It is reported that as many as 40% of the world's insect species, including bees, ants, and butterflies, could become extinct. Such an extreme loss of pollinators is likely to result in insect-pollinated crop plants becoming significantly less productive - further straining already strained global food supplies.

The 2022 UN Biodiversity Conference agreed on an historic package of measures to address dangerous loss of biodiversity and restoring natural ecosystems. The package, known as the Kumming-Montreal Biodiversity Framework" (GBF) includes 4 goals and 23 targets for achievement by 2030. The first of the goals states:
"The integrity, connectivity and resilience of all ecosystems are maintained, enhanced, or restored, substantially increasing the area of natural ecosystems by 2050;

Human induced extinction of known threatened species is halted, and, by 2050, extinction rate and risk of all species are reduced tenfold, and the abundance of native wild species is increased to healthy and resilient levels;

The genetic diversity within populations of wild and domesticated species, is maintained, safeguarding their adaptive potential.".

While the following are among the 23 targets:
i) Effective conservation and management of at least 30% of the world's lands, inland waters, coastal areas, and oceans, with emphasis on areas of particular importance for biodiversity and ecosystem functioning and services. The GBF prioritizes ecologically-representative, well-connected and equitably governed systems of protected areas and other effective area-based conservation, recognizing indigenous and traditional territories and practices. Currently 17% and 10% of the world's terrestrial and marine areas respectively are under protection.
ii) Have restoration completed or underway on at least 30% of degraded terrestrial, inland waters, and coastal and marine ecosystems;
iii) Reduce to near zero the loss of areas of high biodiversity importance, including ecosystems of high ecological integrity;
iv) Prevent the introduction of priority invasive alien species, and reduce by at least half the introduction and establishment of other known or potential invasive alien species, and eradicate or control invasive alien species on islands and other priority sites; and
v) Require large and transnational companies and financial institutions to monitor, assess, and transparently disclose their risks, dependencies, and impacts on biodiversity through their operations, supply and value chains and portfolios.

It can be seen therefore that governments and commercial companies will require biodiversity data to monitor progress, and to take the right decisions to mitigate decline and risk. Currently many governments already have surveying programs in place to monitor general wildlife stocks, protected species, invasive species, agricultural pests, and disease vectors. Many of these survey programs focus on a single species or target group and are designed to manage a particular risk or performance indicator, consequently they do not provide a clear picture of overall biodiversity.

Environmental DNA (eDNA) is a very promising technique to deliver biodiversity data in a cost-effective way. eDNA is genetic material that animals release to the environment. The fragments of DNA or RNA from, for instance, skin cells, scales, and faeces can be sequenced and compared to reference databases to get a taxonomic (species) identification. Samples of eDNA can come from anywhere and are relatively easy to acquire. Unlike current ecological monitoring techniques, that rely for a large part on visual identification of species and, in general, require field surveys and trapping, the eDNA technique is non-invasive and often a lot more sensitive to detect rare and evasive species as well as those organisms that are very small or difficult to identify visually.

Currently eDNA is mostly used in (fresh water) aquatic environments. This is largely driven by the fact that aquatic environments are challenging to survey through conventional methods, but also because fish and amphibians excrete significant amounts of eDNA which is subsequently distributed throughout the water body. Within this context eDNA is often used in ponds or lakes to provide a local but very detailed snapshot. Also, in flowing streams and the ocean where eDNA is used it is assumed the eDNA has its provenance further upstream or is influenced by currents. There are autosampler devices being used to alleviate sampling effort but in many case eDNA collection still requires physical human presence to take a sample. This hampers uptake and deployment at larger regional scales or over significant time periods.

In the paper "Measuring biodiversity from DNA in the air", Current Biology 32, 693-700, February 7, 2022, Clare et al report on a project to filter air samples at a zoological park to collect DNA which was then used to identify species and their ecological interactions. The air samples were found to contain DNA from 25 species of mammals and birds, including 17 known terrestrial resident zoo species. Also identified were food items from air sampled in animal enclosures, and taxa native to the local area - including the endangered Eurasian hedgehog.

In a similar study "Airborne environmental DNA for terrestrial vertebrate community monitoring", Current Biology 32, 701-707, February 7, 2022, Lynggard et al report on eDNA obtained by filtering air samples at the Copenhagen Zoo. Filtering air at three locations (a stable, outside between outdoor animal enclosures, and in the Rainforest House) allowed the capture of eDNA from which were detected 49 vertebrate species spanning 26 orders and 37 families: 30 mammal, 13 bird, 4 fish, 1 amphibian, and 1 reptile species. These species spanned animals kept at the zoo, species occurring in the zoo surroundings, and species used as feed in the zoo. The detected species comprise a range of taxonomic orders and families, sizes, behaviours, and abundances.

Although these two studies showed that air filtering could be used to isolate eDNA, the approaches taken required frequent site visits to change and collect the filter elements on which the eDNA is collected, presenting logistical challenges to widescale deployment.

There therefore exists a need for alternative solutions to the problem of gathering biological material, such as eDNA, from terrestrial animals and other organisms, for use in focused surveys as well as to support investigations into biodiversity.

### Summary

According to a first aspect there is provided a method of generating information about the biology within a survey zone, the method comprising:
i) harvesting biological material from an external impact surface that has been carried through the survey zone by a terrestrial vehicle, the surface having been exposed to air that is displaced by movement of the terrestrial vehicle through the survey zone;
ii) performing molecular analysis on the biological material to generate data on sources of the biological material.

By providing an external impact surface rather than a filter through which air must pass, the approach avoids the need to replace blocked filters. This means that an external impact surface can be exposed to very large volumes of air, and hence the chance to capture more biological material in each survey attempt. It also means that the considerations and limitations associated with filter "pore size" can be avoided: no minimum particle size is defined, and any size of particle may adhere to the impact surface. Using external air impact also avoids the need to provide and maintain a vacuum pump to suck air through a filter.

It is known in the field of air sampling to collect particulate samples on sticky surfaces by impaction due to air currents. Active air samplers (as opposed to passive samplers that gather samples by "sedimentation") may use the principle of inertial impaction to capture particles on sticky surfaces (either an adhesive or something like a culture medium such as agar agar to which particles will readily adhere) as is the case with cascade impactors. In such capture devices an air stream is created using a vacuum source (e.g., a pump) and directed by a nozzle onto the sticky impact surface. Conversely, we are interested in using an air flow created as the result of movement of a vehicle and not a suction source - hence also distinguishing over the approaches adopted by Clare et al and Lynggard et al. We are also interested in capturing samples as deposits on an exposed surface - such a visible exterior surface of or on a vehicle. The impact velocity of particles on our impact surface is a function of the (non-zero) velocity of the vehicle and the velocity of the air (due to weather).

The passage of the vehicle through the survey zone may be under the control of a first party, while the harvesting of the biological material collected on the impact surface may be the responsibility of a different party - for example, the vehicle may be a public transport vehicle or a delivery or haulage vehicle, and the harvesting of the biological material from the vehicle performed by an entity responsible for maintaining a database of biodiversity data, or for providing information on biodiversity to third parties. The approach of piggybacking on journeys that are already being paid for by someone (often someone else) may facilitate extremely cost-effective capture of environmental Nucleic Acid samples, which in turn means that it may become cost effective to sample more widely and/or more frequently, and optionally speculatively. This is an extremely significant advantage of this new approach.

Conversely, the same entity or linked entities may be responsible both for the initial collection phase, in which biological material is captured on an impact surface, and at least the subsequent harvesting phase and possibly the molecular analysis stage. Consequently, according to a second aspect, there is provided a method of generating biodiversity data for a geographical area, the method comprising:
i) collecting biological material on an external impact surface of a terrestrial vehicle moved over the area;
ii) harvesting the collected biological material from the impact surface;
iii) performing molecular analysis on the harvested biological material to generate data on sources of the biological material.

The method according to the second aspect may further comprise creating a database based on the results of the molecular analysis performed in step iii), optionally further comprising populating the database with biodiversity data from multiple iterations of the method, and optionally populating the database with biodiversity data derived from iterations of the method performed for different geographical areas.

In any variant of the second aspect the geographical area may be at least one of an industrial site, a construction site, or a commercial site, the method further comprising collecting biological material on an impact surface of a vehicle moved over one or more geographical zones beyond a periphery of the site, the harvesting and molecular analysis steps also being performed on biological material so collected.

According to a third aspect, there is provided a method of monitoring a geographical area for the presence of a biological marker indicative of the presence of a biological target, the method comprising:
i) collecting biological material on an external impact surface of a terrestrial vehicle moved over a geographic area;
ii) harvesting the collected biological material from the impact surface;
iii) performing molecular analysis on the harvested biological material to generate data indicative of the presence or absence of the biological target.

In the method of the third aspect the biological target may be a rare, threatened, or endangered species.

Alternatively, in the method of the third aspect the biological target may be a species targeted for control or eradication.

In the method of the third aspect the biological target may be a termite, woodboring beetle, or fungi such as dry rot each of which pose a potentially significant threat to the timber used in construction. This aspect of the invention provides a potential early warning system both for timber merchants, importers, and stockists, but also for developers, managers and owners of housing built using significant amounts of timber - for example timber-framed construction. Such an early warning system would enable property owners and managers to make timely and focused interventions to eradicate the relevant pest rather than having to rely on potentially harmful long term preventative use (or frequent "blind" re-application) of insecticides and fungicides - something which has long been associated with chronic illness conditions in people exposed to the chemicals.

According to a fourth aspect there is provided a method of generating information on biological presence within a geographical survey area, the method comprising:
i) collecting biological material on an impact surface of a terrestrial vehicle moved on one or more first transects through the survey area;
ii) harvesting the collected biological material from the impact surface;
iii) performing molecular analysis on the harvested biological material to generate data on sources of the biological material;
iv) performing one or more further captures of biological material on one or more second transects through the survey area, each of the one or more second transects intersecting with one or more of the one or more first transects;
v) harvesting the collected biological material from the impact surface;
vi) performing molecular analysis on the harvested biological material to generate data on sources of the biological material;
vii) generating a probability map of identified species and/or taxa based at least in part on the results of the molecular analyses performed in steps iv) and vi).

According to a fifth aspect there is provided a method of generating a potential occurrence map for biological species of interest within a geographical survey area, the method comprising:
i) collecting biological material on an impact surface of a terrestrial vehicle moved on one or more first transects through the survey area;
ii) harvesting the collected biological material from the impact surface;
iii) performing molecular analysis on the harvested biological material to generate data on sources of the biological material;
iv) performing one or more further captures of biological material on one or more second transects through the survey area;
v) harvesting the collected biological material from the impact surface;
vi) performing molecular analysis on the biological material harvested in step v) to generate data on sources of the biological material;
vii) generating a probability map for biological species of interest based at least in part on the results of the molecular analyses performed in steps iv) and vi).

According to a sixth aspect there is provided a method of generating a probability map of identified species and/or taxa within a geographical survey area, the method comprising:
i) collecting biological material on an impact surface of a terrestrial vehicle moved on one or more first transects through the survey area;
ii) harvesting the collected biological material from the impact surface;
iii) performing molecular analysis on the harvested biological material to generate data on sources of the biological material;
iv) harvesting the collected biological material from the impact surface;
v) performing molecular analysis on the harvested biological material to generate data on sources of the biological material;
vi) performing one or more further captures of biological material on one or more second transects through the survey area, each of the one or more second transects intersecting with one or more of the one or more first transects;
vii) harvesting from the impact surface the biological material collected in step vi);
viii) performing molecular analysis on the biological material harvested in step vii) to generate data on sources of the biological material;
ix) generating a probability map of identified species and/or taxa based at least in part on the results of the molecular analyses performed in steps iv) and viii).

In the method of the sixth aspect the method further comprising dividing the geographical survey area into a matrix of grid cells of unit size, and determining a probability of collection for a given species or taxa for each grid cell based on comparing multiple routes and sample events. Optionally, for each grid cell through which a given route passes allocating an initial probability of collection for a given species or taxa which, in the case that the given species or taxa is detected somewhere along the given route, is equal to 1 divided by the number of grid cells through which the given route passes, or zero or a factor *f* adjusted to reflect a chance of a false negative in the case that the given species or taxa was not detected somewhere along the given route. Optionally, for cells through which a route does not pass a large scale (at the size of the average route length) moving average is applied using the probability of collection of all routes sampled. Optionally, this moving average may be applied for each `probability of collection' grid cell linked to each route, optionally based on other `probability of collection' grid cells through which a route does pass. Optionally, the moving average algorithm could also be another exploration algorithm like Kriging (Gaussian process regression) or multipoint statistics, and/or use secondary data (such as vegetation cover, land use maps, wind velocities, other species occurrence databases) for guidance. Optionally, one average value may be applied for all cells that are further than one grid cell distance from a route, and for all cells within one grid cell distance a "neighbouring" average being the average of all such neighbouring cells (being cells near a route) may be used. Optionally, a base probability is calculated for cells distant from (all) routes based on the mean probabilities of the routes in the (total) survey area. Optionally, at a larger scale, in which some routes are shorter than the total survey area, a moving average may be applied. Optionally, the aggregated/modelled probability of collection for a given species or taxa S 1 (psi) for each grid cell is the product of the probability for that cell for each of the routes considered, meaning that the lack of detection of a given species or taxa on one or more of the routes sets the probability of collection for the cells in which other routes are intersecting that route also to 0 or to f if such a factor is applied. Optionally, for each grid cells next to, but not part of, a route, the mean of the probabilities of an enlarged square around the relevant cell may be calculated to reflect added information available about the area surrounding the relevant grid cell. Optionally, for subsequent sampling moments the probability for grid cells may be recalculated and added to the previous probability map.

Optionally in the methods of the fifth and sixth aspects each of the one or more second transects intersects with one or more of the one or more first transects. In the methods of the fifth and sixth aspects the impact surface may be an external surface.

According to a seventh aspect there is provided a method of monitoring a geographical area for the presence of a disease vector such as the tiger mosquito, the method comprising:
i) collecting biological material on an external impact surface of a terrestrial vehicle moved over the geographical area;
ii) harvesting the collected biological material from the impact surface;
iii) performing molecular analysis on the harvested biological material to generate data on sources of the biological material; and
iv) determining the presence or absence of the disease vector.

According to an eighth aspect there is provided a method of monitoring a geographical area for airborne viral presence, the method comprising:
i) collecting biological material on an external impact surface of a terrestrial vehicle moved over the geographical area;
ii) harvesting the collected biological material from the impact surface;
iii) performing molecular analysis on the harvested biological material to generate data on sources of the biological material; and
iv) determining the presence or absence of a virus based on the results of step iii).

### Brief description of Figures

Embodiments of the invention will now be described, by way of example only, with reference to the accompanying Figures, in which:
Figure 1 is a flow chart illustrating methods according to aspects of the invention;
Figure 2 shows front views of a pair of vehicles that may be used in collecting eDNA and other airborne biological material in methods according to aspects of the invention;
Figure 3 is a map illustrating schematically an idealized transport network utilised for collection of biological samples;
Figure 4 is a map illustrating the determination of detection probability based on findings from surveys performed on intersecting transects;
Figure 5 is a flow chart illustrating a method according to an aspect of the invention; and
Figure 6 is a flow chart illustrating another method according to an aspect of the invention.

### Specific description

In aspects of the invention airborne eDNA (more generally, biological material) is collected on an external impact surface that is moved along a transect (i.e., a route) through a space or area to be monitored using a terrestrial vehicle. The biological material is harvested from the impact surface and then processed, as will be described later. The impact surface may also be referred to as the collection surface. The transect may be several kilometres in length, often more than 10 kilometres in length, and may be tens or hundreds of kilometres in length. Depending upon the length of the transect, the same transect may be traversed multiple times between successive harvesting events, which may occur on the same day or within 12 or 24 hours of each other - although under some circumstances harvesting may occur after only a single traverse of a transect, and successive harvesting events may occur multiple times within a 24 hour period.

Unlike the approaches taught by the Clare and Lynggard papers, where air was pumped through a porous filter so that eDNA is filtered from the air to collect on the filter, we propose the use of an impact surface that is moved through space to cause relative movement between the surface and the air, with air being deflected by the surface and biological material being deposited on the surface as a result of the movement of the surface through space. Rather than using a porous material which captures particles too large to pass through the pores through which air flows, we can use a solid surface on which air flow deposits biological particles without the air needing to pass through the surface. That is, the air flow is deflected by the surface rather than passing through the surface. In this way we can use substantially non-porous surfaces like glass, metal, painted metal, and plastics, as our impact surfaces from which the captured biological material can subsequently be harvested. In this way we avoid the problem of filters becoming blocked and needing to be replaced by a fresh filter. Of course, it would also be possible to provide an impact surface made of porous material, even for example made of a substance that would function as a filter, but used "blind" - that is without taking advantage of air flow through the depth of the filter material.

Our exposed impact surfaces preferably do not present an adhesive surfaces against which sampled particles can adhere - since the use of an adhesive surface is likely to be problematic in situations where sample collection is performed using a vehicle that is also being used in parallel in some other activity such a public transportation or goods haulage.

Our impact surfaces may for example be a window (e.g., windscreen) or body surface of a terrestrial vehicle such as a motor vehicle e.g., a car, bus, truck, or train or may be a cover surface supported by or carried on a major surface of such a vehicle. This approach makes possible the use of impact surfaces whose area is tens or hundreds of square centimetres, or as much as several square metres. The possibility to use impact surfaces of a square metre or more, together with the possibility to maintain average speeds of 60km/h or more potentially for many hours at a time, means that extremely large air volumes can be sampled with relative ease. For example, even assuming an impact surface of just 10cm by 10cm, with an average speed of 60km/h the effective volume of air processed is 10 m³ per second or 3,600 m³ in an hour. This compares with a rate of just 300ml/minute reported in the Clare et al paper, which could be maintained for just 30 minutes (total air volume of 9 litres = 0.009 m³) before the filter needed changing (Clare et al used filters with 0.22µm and 0.45µm pore sizes in the intake end of the tube of a peristaltic pump from Geotech). The Lynggard et al paper reported the use of three air sampling devices - a water vacuum (flowing 8.8 m³ of per minute, for either 30 or 60 minutes, and collecting samples in 1.7 litres of sterile milli-Q water in a vortex chamber), and 24 volt and 5 volt blower fans (flowing 0.8 m³ per minute for 30 minutes, 60 minutes, or 5 hours, and 0.03 m³ per minute for 30 hours, respectively), the latter two using class F8 pleated fibrous filters for airborne particulate matter. No significant differences in detections were found between the three air sampling devices, but practical differences (size, power supply and water supply requirements, noise, etc.) meant that the two blower fans were favoured, despite their lower air flow rates.

Using an impact surface the size of an average city bus windscreen, let's say 2.5m wide by 2 m high, or 5 m², and an average speed of 30km/h we see an effective volume of air processed of 2,500 m³ per second. Even allowing for an assumed lower collection rate of an impact surface compared to a porous filter, there is clearly huge potential in using this approach for eDNA collection. Even with quite a small impact surface, for example 10cm by 10cm or less, useful results can be expected to be achieved by, for example, flying a consumer-level drone (which may easily maintain a speed of between 30 and 60 km/h for as much as 30 minutes) along a transect above a terrestrial zone or region of interest. It will be appreciated that in all aspects of the invention the initial stage of airborne nucleic acid capture could be performed using an airborne vehicle such as a drone or light aircraft, with the impact surface provided on or by the exterior of the airborne vehicle, the exposed impact surface being non-adhesive, albeit that such methods are not currently claimed they remain within the scope of the present invention.

The or each impact surface may be positioned on an exposed frontal surface of the vehicle such that the impact surface can be observed directly by a person positioned in front of the vehicle (at such a distance and height that the entire frontal surface of the vehicle can be seen - which may of course require the person to move themselves or at least their head). But in some applications the or each impact surface may be mounted on an exterior surface of the vehicle that is obscured by another more forward part of the vehicle - for example, an impact surface may be provided on an exterior surface of a trailer or other hauled load bearer that is attached to an HGV (heavy goods vehicle) or other vehicle, the impact surface being obscured by the vehicle's cabin from the view of someone standing in front of the vehicle. Clearly, we want the impact surface to be exposed to air displaced by movement of the vehicle, and when the or each impact surface is carried by a trailer or other hauled entity care needs to be taken to ensure that such exposure occurs despite the presence of the vehicle cab in front of the impact surface and any streamlining or other airflow influencing structures.

If the impact surface is mounted on a terrestrial motor vehicle, this may for example run on a track (e.g. a railway, metro, or tram track), metalled road or unmetalled road or track where average speeds of 50 km/h or 100km/h may be maintained for one or several hours, again enabling huge volumes of air to be sampled with ease, even for vehicles with relatively small frontal areas (e.g. motor cars).

### Airborne particle buoyancy & inertia

The collection of particles of biological material such as eDNA on large external impact surfaces is largely governed by particle inertia. It is not clear how large eDNA particles are, but we assume the DNA largely exist within cells and this is why an appropriate buffer, such as a phosphate buffer, is beneficial when harvesting the eDNA as it mitigates cell burst.

Based on this assumption we expect that DNA or RNA can exist in varying cell/particle sizes. The typical viral particle size is from 0.02 um to 0.5um (but these particles may also be encapsulated in larger aerosols, typically less than 1µm). Bacterial cells may range from 0.2 to 8um and eukaryotic cells range from 5 to 100um. Also, this eDNA may exist in cell clusters but the larger the size of the particle the more likely it is to quickly settle as the density of typical cells material may be around 1000 times higher than air. Forces like drag, buoyancy and gravity work on the particles and within the range of 1 to 100µm we expect settling speeds (the speed with which a particle may move in settling out from the air) of 0.2cm to 15m per minute if there is no wind or other forces acting on the particles.

The heavier the particle the faster the particle will settle and thus be removed from the sampling medium (air). Based on this principle it is thus more likely to sample the smaller viral and bacterial cells via our method. However, this principle is largely counterbalanced by the fact that we require inertia to have the particles impact on the collection surfaces. The larger/heavier a particle, the more likely it is to escape from the diverted airflow and collide with the collection surface. This balancing act between particle settling and inertia is likely the reason that our method is able to collect DNA from smaller cells/particles (virus and bacteria) and larger eukaryotic cells at the same time. This is a unique feature compared to conventional filter-based sampling systems that focus on a specific pore size and cell size, plus filters are prone to clog due to blockage by larger particles.

Of course, forward-facing external impact surfaces on vehicles will on occasion be impacted by flying insects and birds, and also may strike arachnids and "bugs" (e.g., small invertebrates such as ticks, spiders, and insects) that fall from or are suspended by roadside trees and street furniture. There may also be impacts with prey or other food dropped by birds, as well as impacts with faecal matter from birds (perched or on the wing) and possibly also from small mammals resident in roadside trees or street furniture.

Suitable examples of the further processing of collected biological material in order to reveal information about biological presence and, optionally, populations in the area/space being surveyed or monitored will be described later, but first we will give an overview of an exemplary general approach which we have found to be effective for identifying sources of biological material harvested from the air.

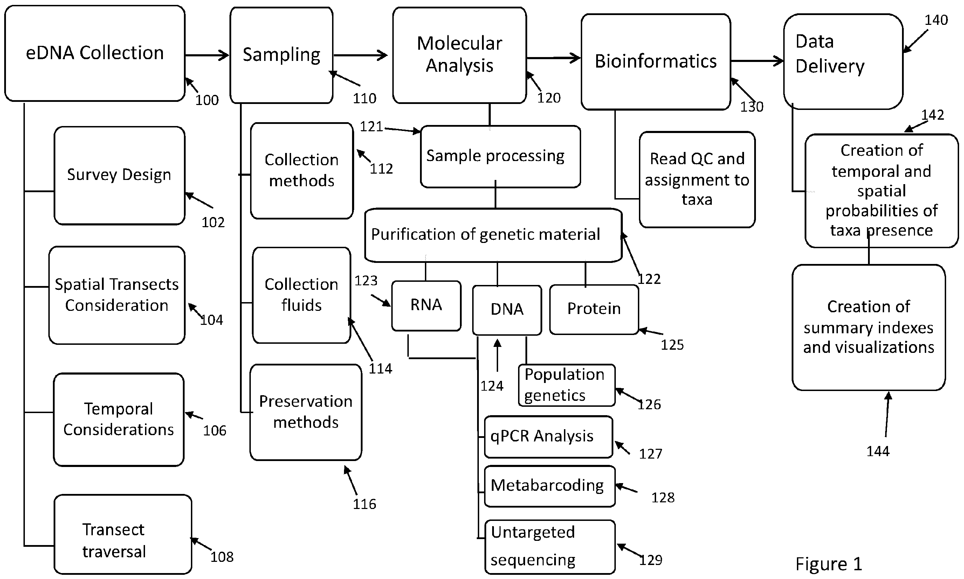

Figure 1 is a flow chart representing elements of possible approaches to methods of processing biological material collected from the air, including various optional elements.

The first stage 100 concerns collection of biological material (here, for brevity generally referred to as eDNA) from the air. Actual collection is performed at step 108 by moving a vehicle along a transect through a sample space to cause eDNA to adhere to an impact surface carried by the vehicle. Generally, the collection of eDNA is preceded by a sequence of planning steps in which a survey protocol is designed 102 taking account of the extent and delimitation of the area or zone to be surveyed, and also of the survey's objective in terms of the details of what (in a biological sense) is being looked for. For example, whether one or more identified species or taxa are being sought (either to confirm presence or absence), or whether a more general biodiversity survey is to be performed.

At step 104 consideration is given to the accessibility of the area to be surveyed, in particular the extent to which transport routes (e.g., roads, tracks, paths, railways, tram lines, waterways, etc.) traverse the area, and also the extent to which such transport routes are traversed by vehicles. If an area to be surveyed is too sparsely covered by suitable transport routes it may be necessary to consider the use of one or more aerial vehicles, such as drones, microlights, or light aircraft as collection vehicles. Such a situation is most likely to arise in more remote, rural areas, but aerial vehicles may be useful for ensuring adequate cover of a large survey area even though the area is close to an urban area. For use as a collection device a suitable aerial vehicle may be provided with an external impact surface that is non-adhesive (in the sense that the collection surface is non-adhesive) surface to which particles adhere in use. The external impact surface may be a surface especially added to the vehicle, such as a glass or plastics sheet, or may be an already-present vehicle surface.

At step 106 account is taken of temporal considerations - potentially both with regard to the nature of the target biology (e.g. whether the target biology is a nocturnal or diurnal species - which is likely to be more relevant for insects such as moths than to large mammal species such as badgers, and seasonal behaviours such as hibernation and reproduction) and also the relevant transport hours if advantage is to be taken of a potential host vehicle or vehicle category (public transport, delivery or haulage vehicles, for example). Account may need to be taken of time of year (summer or winter), public holidays, days of the week, school term times, etc., since each of these are likely to affect the timetabling (including routes taken) and frequency of public transport, and may also affect freight transport routes, and frequency. The timing of scheduled stops for vehicles, e.g., for cleaning, change of driver, public transport hub and terminus arrival/departure times, may also need to be taken into account to ensure efficient and economical collection and harvesting of eDNA.

The second stage 110 biological material is sampled after having been collected on the collection surface (also referred to as an impact surface). Collection or harvesting 112 may be performed for example by swabbing (e.g. using a roller, swab, sponge, wipe, typically pre-treated with a suitable collecting fluid 114) or rinsing the collection surface (for example if the collection surface is an element of the vehicle that has made a transect through the sample space) with a suitable collecting fluid 114, or by first removing the collection surface from the vehicle (for example if the collection surface is a dedicated surface that has been added to, e.g. is carried by but is readily removable from, the vehicle) and then processing, e.g. washing the collection surface with a suitable collecting fluid 114, possibly after segmenting the collection surface into multiple pieces. The harvested biological material, possibly while still attached to a collection surface removed from the vehicle, or on a roller, swab, sponge, wipe, or filter, may then need to be preserved 116 in order to maintain the integrity of the biological material (e.g. DNA, RNA or protein) for example by placing the material in a temperature controlled environment (e.g. in a refrigerated environment) and/or by adding a preservation buffer which may allow samples to be stored and transported at ambient temperatures without degradation.

Subsequently, and typically within a suitable laboratory having a controlled environment to reduce the risk of cross-contamination, the samples are subjected to molecular analysis 120. The samples are initially processed 121 and prepared for purification of DNA, RNA, and/or proteins or other biological substances. Typically, this will involve washing out sampling matrices (rollers, swabs, filters, sponges) or washing demounted collection surfaces with a buffer such as a phosphate buffer (or other suitable liquid) to collect the acquired biological and cellular material. Biological and cellular material may then be concentrated from the liquid (buffer) suspension by centrifugation. RNA 123, DNA 124, or proteins 125, for example, may then be purified 122 from concentrated pellets using commercially available reagents and methodologies, for example as will be further described later.

Extracted DNA may be subject to population genetic studies 126. Extracted DNA and/or RNA may be subject to qPCR analysis 127, metabarcoding 128, and/or untargeted sequencing 219. Typically, we are not interested in collecting or analysing human DNA: the focus is upon the extraction of non-human DNA/RNA/proteins from the biological material that is collected. In particular, we are not generally interested in processing to isolate markers characteristic of individual humans. Nevertheless, there may be situations where it is useful to collect human DNA for analysis, and it may on occasion even be chosen to isolate markers characteristic of individual humans.

It will also be appreciated that we may be interested not only in identifying animals but also in identifying plants and/or fungi - for example we may be interested in identifying the presence of invasive or problematic plant species, such as Japanese knotweed.

Bioinformatics processing is then applied 130 to the results obtained at the molecular analysis stage 120 optionally to determine aspects such as genetic variability, derive all identified species, determine relative species abundance, or characterize the genomes and/or derive an organism's (potential) activity. The use of RNA or DNA to identify functional or active genes, independent of the organism's taxonomic identification, can tell us various things about the environment. Especially for bacteria this can help us tell if there are more genes detected to, for instance, facilitate nitrogen cycling in one area versus another area. Quality filtering 132 is typically applied followed by denoising and assigning reads to taxa.

The fifth step 140 involves data delivery, the first step 142 of which is the determination of temporal and spatial probabilities of taxa presence. Thereafter, summary indexes and visualisations may then be prepared at 144.

For the metabarcoding lab analysis the bioinformatics process is done following the best practices from the literature and delivers a set of quality-controlled (QC) reads - the output of the sequencing machine, consisting of nucleotide strings and associated quality scores; they are not necessarily unique in a sample, an inference of the unique DNA or RNA sequences present in the collected sample that are amplified by the used primer pair and, as the final output, a table with the taxa counts (number of reads assigned to each taxonomic group), and a measure of the signal intensity for each taxa in each sample. The specific methods to achieve each of these incremental results are not necessarily dependent on each other and can be substituted later. Suitable methods will be discussed later.

If the laboratory procedure used is shotgun (also called whole genome) metagenomics, then the bioinformatic analysis needs to be adjusted accordingly following best practices from literature to include:
visualization and trimming of reads based on their quality scores;
assignment of reads to genomes;
identification of predicted gene sequences; and
mapping of predicted gene sequences into detected protein domains.

If the laboratory procedure used is population genetic analysis, then the bioinformatic analysis needs to be adjusted accordingly following best practices from literature to include: identification of molecular markers from raw sequencing reads; and estimation of number of individuals from the genetic diversity observed in the molecular markers.

It will be appreciated that the described approach to the collection of biological material from the environment is applicable not only to an assessment of biodiversity but also to survey programs focused on a single species or target group, whether the species or group is monitored for reasons of conservation or because the species or group is considered a risk. That is, the techniques described may usefully be applied to detecting the presence of known rogue/non-native species, known pests or animal/plant vectors, as well as at-risk, endangered or vulnerable species, etc.

Having set the scene, we will now consider aspects relating to the initial acquisition of biological material by the movement of a collection surface along a transect through a sample space. Later we will discuss the issue of transect definition, selection, and the timing and location of sample harvesting.

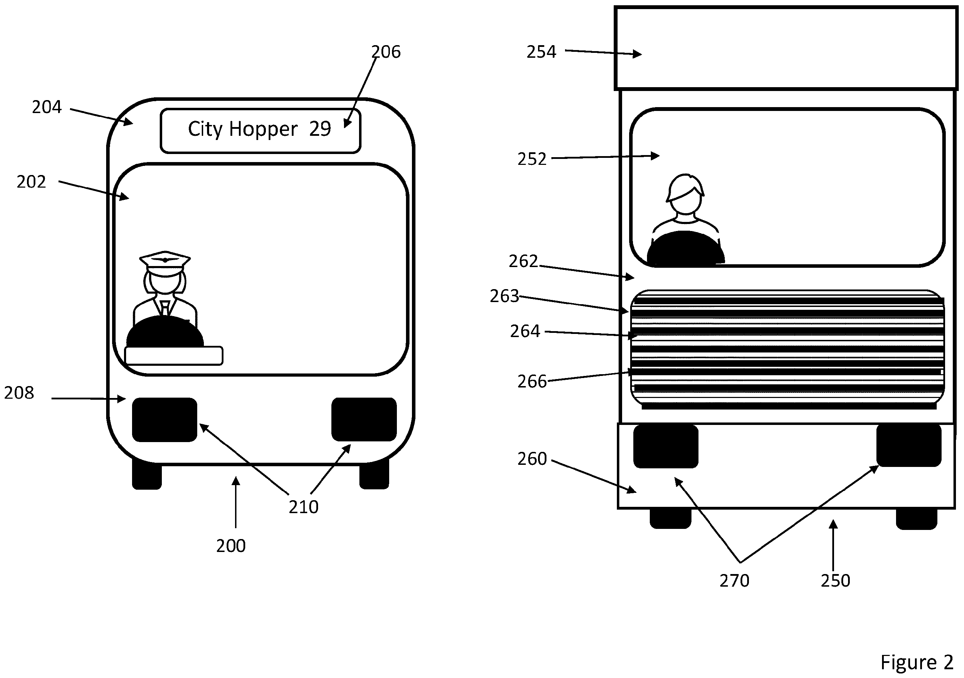

Figure 2 shows in simplified form views of the fronts of a pair of vehicles, a city/transit bus 200 and an HGV truck 250, which are examples of larger vehicles that may be used to perform transects to acquire eDNA from an area to be surveyed (smaller vehicles, with smaller frontal areas, such as light delivery vehicles and cars may also be used in this new technique). These two vehicles are wheeled land vehicles, other examples of which are trains and trams, but other terrestrial vehicles such as water vehicles (e.g., boats and other water craft), and non-wheeled land vehicles (e.g., tracked vehicles, hovercraft) may all be used in the gathering of airborne biological material, as may aerial vehicles (such as light aircraft and drones, as discussed elsewhere).

The bus 200 will typically have an overall height of around 3 metres (e.g., 2.99 metres) and a width of about 2.5 metres (2.55 metres is typical). Typically, the front face of the bus 200 is generally planar, often being arranged to be vertical or nearly vertical in use, with a glass windscreen 202 that extends across virtually the whole width of the front face, and that typically has a vertical extent of at least 1.5 metres. Above the windscreen 202 is a body panel 204, which is typically generally planar and which typically, within which may be located a glass panel behind which may be mounted an indicator array 206 to indicate a destination, route, and/or route or bus number. Beneath the windscreen 202 is another, typically planar, body panel 208 which may contain headlights 210.

The truck 250 will typically have a width of about 2.5 metres and an overall height of about 4 metres (with a range from roughly 3.75 metres to about 4.2 metres). As with the bus 200, the front of the truck 250 may be generally planar, although there tends to be a greater variation in truck cab design than in the design of the front ends of city buses. The windscreen 252 of the truck 250 again extends across virtually the whole width of the front face, and typically has a vertical extent of at least 1.5 metres. Above the windscreen 252 is a body panel 254, which may be generally planar and may be disposed vertically or which may slope back to reduce wind drag or simply for reasons of aesthetics. Typically, the lower end of the front of the truck 250 includes an impact absorbing bumper arrangement 260 that, for reasons of pedestrian safety may have a generally flat front face, typically with a vertical extent of between 30 and 60cm or more. Between the bumper arrangement 260 and the lower edge of the windscreen 252 is a further body panel 262 which may include a grille 263 having bars 264 separated by gaps 266 through which air can flow, for example to one or more radiators (heat exchangers) mounted behind the grille 263. The bumper arrangement may include a pair of headlights 270.

When either of these vehicles 250, 252, is driven through an open space such as a city or a natural environment, eDNA (and other biological material) from the environment will be deposited onto the vehicle, in particular on the front of the vehicle. Our experiments have shown that this biological material tends, under normal circumstances, to remain on the surface of the vehicle so that it can subsequently be harvested from the vehicle's surface. We have successfully harvested biological material from the fronts of such vehicles by means of paint rollers (of the types used by decorators to apply paint to the walls of buildings, sponge, short pile or long pile "hairy" fabric rollers of synthetic material are widely available in a wide range of sizes- so that a suitable sized roller can be selected according to the size and shape of the vehicle part being treated) which are used to swab the vehicle surface. In general, we favour the use of "hairy" rollers (either short pile or long pile) as it is easier to wash the biological material out from such rollers than from sponge rollers. Short pile rollers are useful because their smaller diameters make them more suitable for collecting biological material from non-continuous surfaces such as grilles, gaps around headlights, etc. If the paint rollers are pre-soaked in a suitable buffer, such as a phosphate buffer, prior to sample collection it tends to increase the adherence of the biological material to the matrix of the roller. We have also successfully harvested biological material using synthetic fabric swabs, and acrylic sheets (including A4 overhead projector transparency material) adhered to the windscreen of a bus. The acrylic sheets were removed from the vehicle after the collection phase, and then taken to the lab for the harvesting phase. Pre-soaking the fabric swabs in a suitable buffer (e.g., a phosphate buffer) prior to sample collection tended to increase the adherence of the biological material to the matrix of the collecting device.

Experiments performed on municipal buses have also shown that eDNA (and other biological material) in practice adheres particularly well to glass surfaces - such as the windscreen 202 and the glazed panel for the indicator array 206. Additionally, our results indicate that valuable biological material can be collected from windscreens 202 even after being swept by the vehicle's windscreen wipers (and washers).

It is possible that significant amounts of biological material may remain adhered to the windscreen wipers (blades and or frames) even after the vehicle is cleaned, consequently it may be decided to avoid taking samples from the windscreen wipers unless the presence of material captured during earlier journeys is not a problem - e.g. based on routes traversed and the goal behind the harvesting of biological material.

As well using what might be termed the native surfaces of vehicles as impact surfaces for collecting biological material from the air, the large frontal areas of vehicles such as buses, coaches, delivery vehicles, trucks, trains, etc., means that it should be possible to find space for a demountable collection surface that is usefully large (e.g. 100 cm² or more). Removable, optionally self-adhesive, plastics films (e.g. vinyl or other suitable materials) may be applied as a wrap to body panels or sections of body panels and then removed with collected biological deposit intact. This approach would skip the surfaces cleaning (sterilization) steps prior collection to remove previous DNA traces and would ensure that the biological information was really collected during that specific sampling trip.

Alternatively, a roll of plastics film may be used with a mechanism that moves film from a supply to a take up device, the film being stretched across part of the front of the vehicle to provide an impact surface that is progressively refreshed by winding the film from the supply to the take up device. Preferably the film is supported, for example by the relevant part of the body so that the film is not free to flap under the influence of air flowing over the vehicle as the vehicle moves. To harvest the collected biological material the used film is removed from the vehicle for processing in the laboratory. Such an "active" impact surface could include a marking arrangement that time stamps and/or location stamps the film so that it becomes possible to identify a position along the length of the film that corresponds with a known time and/or location ( a vehicle's navigation system, e.g. satellite navigation system, may record details of the vehicle's journeys so that it becomes possible to know where the vehicle went, when it was in any particular location, and how long it spent at any location). It can also be beneficial to collect atmospheric data, such as temperature, humidity, etc., optionally using a suitable on-board weather device (based on a suitable chip) or some external source.

A mechanism could be provided to move plastic film across an impact area that is positioned between a reservoir or store of film to a take up device, with the take up device arranged to remove an exposed section of film from the impact area after a certain period of time (or after a certain distance travelled), with a fresh length of film being advanced from the store into place at the impact area. Optionally the take up arrangement could be arranged to advance a sufficiently long length of film after each "exposure" that the exposed film when would in the take up device is not able to transfer its captured biological material to either face of the film at the location of another "exposure". It will be appreciated that if this is not done there is a risk that captured biological material on the exposed face of the film may be transferred to the back of another (adjacent) length of film - so that a single "frame" of film may then contain biological material from two or more exposures spread across its front and back surfaces. Preferably also there is a system of marking the film (pre or post exposure) so that the film can be cut into individual exposures that can then be processed separately to provide information about the biological material gathered during the relevant exposure of the film. By synchronising exposure periods with location (a transect or a section of transect) based for example on navigation system data, tracking device data, or way point data, it becomes possible to obtain a series of "biological snapshots" that can be mapped to real world locations. Such an approach is particularly useful for biological mapping using vehicles that do not necessarily follow a regular route between pairs of locations (e.g. vehicles that, according to traffic density and accident reports may use the M11, M1, A1, or M40 to drive from London to the English midlands), particularly vehicles that are driven between locations that are very remote from each other - e.g. road haulage vehicles, rather than typical public transport vehicles. Such an approach would also potentially be of significant benefit if using an aerial vehicle to perform sample capture, as it would enable samples from different transects to be kept separate by using a new "exposure" for each new transect, permitting extended capture periods while retaining the capacity to analyse separately the results from different transects. The use of solar-powered drones with such a multiple "exposure" capture mechanism would be one way to reduce the cost of performing capture using aerial vehicles (quite possibly the biggest drawback to aerial vehicle capture).

As an alternative to plastics films, other demountable collection surfaces may be mounted on a vehicle for collecting biological material such as eDNA. For example, FTA (Flinders Technology Associates) cards may be mounted on the vehicle (e.g., on a front panel or windscreen of the vehicle) to capture airborne biological material as air flows past the moving vehicle (such cards may be damaged by rain or prolonged exposure to sunlight, so this needs to be borne in mind when considering how and when to use them in this application). Similarly, panels or other formats of material may be mounted on the vehicle to provide a demountable impact surface - for example panels of metal, plastics or even glass. Indeed, the discovery that airborne biological material adheres well to glass substrates means that the use of demountable glass panels as impact surfaces is attractive. The glass screen protectors (made for example of Gorilla Glass (RTM)) that are widely available for smartphones and tablet computers are extremely tough, and the largest, for example those available for Apple Corporation's iPad Pro (RTM) with a 12.9 inch (32.77cm), have a surface area of over 500 cm². Such glass panels could be held securely in suitable holders, for example clamped into place in a frame that secures the periphery of the glass panel, the frame being securely attached to the structure of the vehicle. Suitable holders may be semi-permanently secured to a body panel of the vehicle, for example using threaded fasteners that screw into captive nuts inserted into bodywork of the vehicle, and the impact surface clipped or clamped into place in the holder using an over-centre or other quick release clamping arrangement. Preferably, if the impact surface is provided by a glass sheet, the holder is configured to support not just the periphery of the sheet but also to provide a (preferably) resilient backing arrangement - for example using foamed or sponge rubber of plastics (e.g. neoprene) to reduce the risk that the sheet will be shattered as the result of impact with a flying projectile such as a stone. Such a backing arrangement may not be possible where the sheet is mounted so as to cover an underlying display surface or other surface (such as the lens of a light or indicator) without significantly reducing visibility of the underlying surface.

Rules on pedestrian safety may preclude the mounting of the panels at a level lower than about 2 metres from the ground, but the panels could be mounted above the upper edge of the windscreen 202, 252, for example on the panel 204 or 254. Given the high transparency of the glass panels, it would even be feasible to mount them in front of the glazed panel for the indicator array 206 without significantly impairing the visibility of the indicator panel. Clearly such glass panels could readily and conveniently be mounted on other vehicles, both large and small, and they do not require coating with something like an adhesive or culture medium in order to capture and hold airborne nucleic acid. Such panels would even be suitable for mounting, as capture surfaces, on lightweight aerial drones provided suitable consideration is given to the panel(s)'s impact on the drone's aerodynamics and flight behaviour. After the vehicle has made one or more transects of the space to be surveyed, the glass panels could be removed for laboratory harvesting of the biological material or the material could be harvested with the panel(s) still in position on the vehicle.

Vehicle grilles, such as 263, will often provide suitable collection surfaces - e.g., the bars 264 of the grille, which are directly exposed to airflow consequent on the vehicle's movement. Biological material may conveniently be harvested from the grille using small paint rollers (e.g., those available with lengths of between about 2.5 cm and 15 cm).

Preferably, before traversing a transect to acquire biological material, the collection surfaces are cleaned (if they are not already clean). For example, cleaning may be done using a 10% bleach solution. The bleach solution being applied and allowed to act for 2-3 minutes. After bleaching the surface should be thoroughly rinsed with distilled water and allowed to dry. 70% ethyl alcohol solution may be applied to speed up drying. Additionally or alternatively, because in field cleaning is unlikely to be 100% successful in removing DNA from surfaces or other parts of collection devices, it is also possible to collect (sample) biological material already present before the device is cleaned and then to consider these previous way points when interpreting data subsequently obtained from a cleaned device or surface.

All the matrices used for collection of biological material should be cleaned and clear of contaminant DNA. Cleaning may be done using 10% bleach solution followed by thorough rinse with distilled water or by a suitable autoclave cycle (with high temperature conditions) if the material of the matrix and casing permits. Swabs, rollers, filters and sponges are preferably pre-soaked in a suitable buffer, such as a phosphate buffer, prior to sample collection, in order to increase the adherence of the biological material to the matrix. The collecting devices may be individually packaged in sealed containers to maintain them free from contamination (preferably the collecting devices are sterile and maintained in this state by suitable, ideally individual packaging).

After collection, samples are then suitably preserved in order to avoid degradation of the biological material and to maintain the integrity of any DNA/RNA proteins. For example, samples may be refrigerated and maintained at a temperature around 4°C, including during transportation. In specific cases (for example, samples in conical tubes) a preservation buffer may be added allowing the sample to be stored and transported at ambient temperature. Removable impact/collecting surfaces may be transported intact to the laboratory where they are subject to processing to harvest the gathered biological material - for example by swabbing, washing, or the like, or these processes may be performed outside the laboratory and the resultant samples then sent to the laboratory.

Collected samples, suitably labelled, are then subject to sample processing. As a first step the relevant biological material (DNA, RNA, protein) is extracted and isolated from the sampling device. In the laboratory samples are processed and prepared for, for example, purification of DNA or RNA. Different matrices are be subjected to different procedures/protocols. Examples of suitable such protocols are set out here:
1. Rolls, sponges, filters, cones, plastic / charged surfaces collected in whirl-pak bags are washed out with freshly prepared phosphate buffer saline (PBS) (1L prepared by adding 8 g of Sodium chloride; 0.2 g of potassium chloride; 1.44 g of sodium phosphate dibasic and 0.245 g of potassium phosphate monobasic. Adjust solution to pH = 7.4). After washing out the matrix, biological and cellular material is concentrated from buffer suspension by centrifugation. Pellets are then used for extraction with a commercial kit Qiagen DNeasy ^{®} Blood & Tissue kit (Qiagen) following the manufacturer's instructions (example Reference: Clare EL et al., eDNAir: proof of concept that animal DNA can be collected from air sampling. PeerJ. 2021 Mar 31;9:e11030. doi: 10.7717/peerj.11030. PMID: 33850648; PMCID: PMC8019316)
2. Air dried FTA Cards are cut into pieces and placed in a 2mL micro tubes. Swabs are also collected in micro tubes. DNA from FTA cards and swabs is extracted using with the same commercial kit Qiagen DNeasy ^{®} Blood & Tissue kit (Qiagen) following the manufacturer's instructions. (example References: Deiner K, et al., Choice of capture and extraction methods affect detection of freshwater biodiversity from environmental DNA. Biological Conservation. 2015;183: 53-63.; Tsuji S, et al., The detection of aquatic macro-organisms using environmental DNA analysis-A review of methods for collection, extraction, and detection. Environmental DNA. 2019;1: 99-108.)

Having extracted and isolated the relevant biological material(s), e.g. DNA, RNA, protein, analysis is performed.

In the case of DNA and/or RNA the analysis of the purified genetic material is done using a specific technique, depending on the question asked.
i) For the targeted detection of a specific species or taxa we use PCR to analyze DNA:
   a. real-time PCR (technique in which the amplified DNA product (or amplicon) can be recorded as the reaction progresses, in real time, with product quantification after each cycle, based on fluorescence detection)
   b. or digital PCR (a specialized approach to nucleic acid detection and quantification that estimates absolute numbers of molecules through statistical methods. In this approach the PCR reaction mix is digitized into 20-30 thousand nanolitre-sized microreactions. As this digitization process distributes the PCR mix across so many microreactions, each microreaction will effectively either contain one, zero, or just a handful of the target nucleic acid molecules. The isolated microreactions are then amplified, and data are collected from each microreaction at the end of the thermal cycling process. Microreactions that do not contain the target will not show post-amplification fluorescence, while those that do contain the target will show post-amplification fluorescence.)

This approach requires that we use a pair of primers (DNA sequences between 18 and 24 bases in length) that must specifically target the DNA of the species or taxa of interest, and unambiguously discriminate them from taxonomically close relatives.

Depending on the target of interest, we may use primer sequences previously described in the literature or design our own primers, using the reference sequences published in public databases (ex, Genebank; EMBL; SILVA, etc.). We have had good results with the following primers: two different 16S targets and COI. Generally, we have found that using more than one primer can be beneficial, as each primer may reveal the presence of species not revealed by other primers. So, for example we have found more extensive population information through the use of the three named primers than were revealed by the use of any one or two of the primers.

PCR reagents are commercially available from the leading companies in molecular biology products: Thermo Fisher, BioRad, Roche, etc. PCR can also be used to analyze RNA in specific contexts. RNA analysis can be done to identify and quantify the expression of specific genes that may be indicative of organism activity, resistance (ex. to a pollutant, to antibiotics, etc.), etc. The first step of RNA analysis is conversion of RNA to DNA using standard reagents that are commercially available from the leading companies in molecular biology products: Thermo Fisher, BioRad, Roche, etc.

After conversion of RNA into DNA the same procedures and reagents as described above can be used.
ii) Metabarcoding Analysis of DNA or RNA. Metabarcoding is the tagging (barcoding) of DNA/RNA in a manner that allows for the simultaneous identification of multiple taxa and/or organisms within the same sample. This analysis is used for example to characterize insect diversity in a certain green area, or the community of bacteria (microbiome) in a soil sample, or the species of marine mammals and fish in a defined marine area.

Barcoding of one particular sample is done by combining one unique barcode sequence with one primer sequence specific for a certain taxa/organism of interest. Multiple unique codes are available allowing the tagging of multiple samples and multiple taxa/organisms of interest. Barcoding is done by PCR and the taxa/organism-specific primers used for metabarcoding are widely described in the literature.

In the laboratory the chosen primers are verified for specificity and performance in the PCR. PCR conditions change according to the primer sequences and may change according to samples type. These optimizations are often empirical. PCR reagents are commercially available from the leading companies in molecular biology products: Thermo Fisher, BioRad, Roche, etc.

The DNA products of the barcoding originated by PCR (libraries) are then analysed by next generation sequencing (NGS) using one or more high-throughput sequencers. Suitable sequencers are available from Illumina, for example the MiSeq and NextSeq models (differing in analytic capacity and output). Suitable technical protocols for the analysis pipeline starting from a DNA sample and ending up with the analysis of a DNA library by NGS (Amplicon sequencing) are available from the manufacturers of the high-throughput sequencers (e.g., from Illumina).

Biological material, such as eDNA, collected as described above may also be the subject of untargeted DNA or RNA NGS analysis. Untargeted NGS analysis does not use specific primers to amplify samples during the library building process. Thus, the DNA and/or RNA in the entire sample can be sequenced without previous knowledge or selection of the taxa/organism to analyze. This analysis is done when one does not know a priori the organism that may be present in a given sample. Suitable commercially available reagents and corresponding technical protocols to process DNA samples and generate DNA libraries for NGS analysis are available from the usual suppliers (and are described in the literature).

Following the lab analytical process, the data generated may be processed bioinformatically to determine genetic variability, derive all identified species, determine relative species abundance or characterize the genomes and/or derive an organism's (potential) activity.

For the metabarcoding lab analysis mentioned in the previous section, the bioinformatics process delivers a set of quality-controlled reads (the output of the sequencing machine, consisting of nucleotide strings and associated quality scores; they are not necessarily unique in a sample), an inference of the unique DNA or RNA sequences present in the collected sample that are amplified by the used primer pair and, as the final output, a table with the taxa counts (number of reads assigned to each taxonomic group), a measure of the signal intensity for each taxa in each sample. The specific methods to achieve each of these incremental results are not necessarily dependent on each other and can be substituted later. Exemplary suitable methods include the following:
Sequencing quality filtering: due to the nature of the PCR amplification used in metabarcoding analysis, the correct output reads of the sequencing machine will include the sequences of the primers used for amplification. To exclude other reads, read-filtering software such as Cutadapt is used to detect this primer sequence in the reads of each of the sequencing libraries. Reads (or read-pairs when paired-end sequencing is used) which do not contain the sequences belonging to the primer pair used for amplification are removed from the rest of the analysis (allowing for some sequencing errors). Afterwards, reads which fall below a specific quality (Phred score, a metric of confidence reported by the sequencing equipment that can be converted into a probability of error) threshold or that have a number of expected errors above another threshold are filtered out. These thresholds can be manually defined or estimated from the data using appropriate software.

Inferring unique sequences in sample: the general process in this step is called "denoising". It consists of an evaluation of the sequences in each read and their associated quality scores, followed by a grouping of those reads that, given the estimated error probability (from the Phred scores described above), are likely to represent the same original sequence. Several denoising software packages are available, e.g. DADA2. When using paired-end reads, software packages such as DADA2 also perform the necessary trimming and merging of the read pair. The required parameters (position at which the reads are truncated, resulting length of the merged reads) can be manually defined or estimated from the data. A sensible approach is to try the automated estimation first and, if the results are not ideal, the parameters may be manually set based on previous experience with the primer-pair used for the amplification of the samples.

Assigning reads to taxa: this step in particular can be done in a variety of ways. For example, using a naive Bayes classifier approach as is implemented in Qiime2. Naive Bayes classifiers are a family of probabilistic classifiers that, among other applications, are used for text classification. They are also used for assigning taxonomic information to eDNA sequences. Classifiers are created by training on a set of sequences with known taxonomic annotations. Optionally, these classifiers may be trained on the sequences that are expected to be amplified by each set of primers. These expected sequences are first checked for uniqueness and ambiguity in case the same sequence can match more than one species. Upon sample sequencing, the classifier created for the primer pair used in each library is applied to the sample's inferred sequences identified in the previous step. The classifier outputs a taxonomic assignment for each sequence above a chosen confidence level (usually 95%). If the classifier cannot reach a species-level identification above the confidence level, it attempts to report a genus-level classification above the confidence level, and so on, moving up in the taxonomic classification.

The analysis can be redone starting at each of the steps. The identification of the taxa associated with each sequence, for example, can be redone at a later stage to take advantage of more complete databases. This allows for a continual improvement of the data even without new samples. The metabolic or ecological functions associated with each taxa can also be improved as new data (both internal and external) is added at a later stage. For example, a species that was detected at a previous point in time can be later discovered to be a key pollinator.

If instead the laboratory procedure used is shotgun (also called whole genome) metagenomics, then the bioinformatic analysis needs to be adjusted accordingly. This will require:
Visualization and trimming of reads based on their quality scores via software such as FastQC and Trimmomatic;
Assignment of reads to genomes using software such as mOTUs2;
Identification of predicted gene sequences;
Mapping of predicted gene sequences into detected protein domains from databases such as Pfam, InterPro or KEGG.

From the identified protein domains, it will be possible to better characterize the metabolic potential of the samples.

A third laboratory technique for processing the samples is a population genetic analysis. This technique identifies molecular markers for individuals, such as single-nucleotide polymorphisms (SNPs), precise locations of the genome where the specific nucleotide (or letter) of the DNA might vary between individuals. This will require:
Identification of molecular markers from raw sequencing reads;
Estimation of number of individuals from the genetic diversity observed in the molecular markers.

We will now consider the issue of determining a collection program, including transect selection. The collection of the aerial DNA along a transect can be performed through aerial DNA capture using various vehicles. In many cases, the transects along which these vehicles move are governed by operational limitation like public bus-routes, rail networks, ferry and shipping lanes, delivery vehicle networks, trucking schedules, general road access and, for instance, air restriction for drones and other aerial vehicles.

The design of a sampling transect is determined by the survey objectives and the operational limitations. The following three survey objectives are likely to be relevant to many different transect design considerations:

### i) Continuous* survey of aerial DNA in a region or country

To survey aerial DNA (i.e. biodiversity, detect target taxa or even determine populations) at a significant scale of a region or country (range of >10000 Km2) it is required to acquire enough samples to resolve the required spatial and temporal resolution and significantly lower survey costs. To achieve this, it is seen as most prudent to utilize existing transportation networks (i.e. public bus-routes, rail networks, ferry and shipping lines, delivery vehicle networks, etc.). In this situation it is thus helpful to understand thoroughly these routes and their time-schedules to allow for sample acquisition (harvesting) during prolonged stops or at (overnight) depots. In any case, the transects will preferably together collectively cover (at least cross) the entire area to be surveyed (or at least the bulk of it, so that additional sample gathering journeys sufficient to cover the rest of the area need not be very numerous or difficult to arrange), intersecting each other and providing substantial duplicate sampling opportunities (i.e. transecting the same or closely adjacent locations multiple times). In many situations it is likely that logistical constraints and road networks hamper an ideal survey design. An ideal survey design would be a set of orthogonal perpendicular and straight transects spaced in such a way that they mimic the required spatial resolution. In this idealised situation each transect only intersects another transect once and the distance between lines is stable and easily resolved in further computational steps.

In the real world there are two critical aspects to consider that cause significant deviation from this ideal orthogonal & perpendicular set of intersecting transects: i) road networks and public transport routes tend to be more densely spaced in populated (urban areas) and converge there, ii) lines are not straight and may thus intersect each other in multiple occasions or even run parallel over significant distances. These two complexities can partly be resolved computationally if there are enough transects and sampling events. In addition, output maps can be computed in a way that they portray uncertainty in detection (e.g. the measure of the likelihood of detection/presence of a taxa in a mapped grid location) as well as a varying spatial resolution (size of the mapped grid location). To meet survey objectives, it is thus important to understand the computation possibilities in each survey design and marry them with logistical and operational possibilities. Furthermore, it is important to collect the timestamped coordinates or routes along which these vehicles have travelled during sampling.

Geographical ^{∗}continuity may be the goal, but it can also be interesting to perform surveys that are quasi-continuous in a temporal sense too (although the use of transects performed by public transport vehicles may inevitably introduce temporal gaps in survey data gathering.

### ii) Specific comparative study of aerial DNA over space or time

In a situation where one wants to use this technology to compare two or more measurement of the aerial DNA (i.e. taxa in an area (e.g. same location, different times) or compare taxa in two or more different areas, it is extremely helpful to limit variation in the eDNA collection methodology. This means that the same or similar vehicles or collection devices/methods are preferably used to gather the material samples from the air. It will also be helpful if elements like speed of movement, time of day and weather conditions are the same or very similar.

In some situations, rail or road layout or access limitations will govern the possibility to sample consistently between sites or over time. In such cases, these variations should be captured closely through collection of maps, travel speeds and time schedules, weather conditions, vehicle types and collection methods/devices, to enable an optimal collecting schedule and methodology to be determined.

To survey an area effectively and generate realistic measurement of organisms' presence via aerial DNA, it is often required to run a transect multiple times or along various routes to acquire multiple samples. Environmental DNA has an inherent stochastic nature mostly linked to the source organism's ecology and physiology and unknown persistence rates of (air) DNA molecules to environmental elements (UV exposure, dryness, humidity, etc) and dispersion capabilities from origin. It is hence helpful to enhance capture potential of true positives and limit risks of false positives by the use of multiple eDNA collection events.

The data delivered by such a study can, if done correctly (i.e. collection device/method variations between measurements very limited and statistically robust sample numbers) provide relative abundance of aerial DNA and may potentially be used to infer (relative) species abundance, and the likelihood for their presence or absence.

### iii) Detection of a target taxa or group

In a situation where one merely needs to detect a specific aerial DNA sequence it may be sufficient to limit survey effort only to the main location. In such cases, it is important to work with both positive and negative controls in the laboratory as there is limited contrasting sample data. It is also recommended to acquire clear negative and positive field controls within or around the focal site, using the same sampling approach, as well as to collect multiple biological replicates (i.e. environmental samples that are taken in the exact same conditions or in parallel) to reduce the likelihood of false positives.

Seasonal and weather conditions may also influence species activity levels, DNA shedding rates and dispersal. Therefore, collection in multiple seasons, times of day or weather conditions is likely to be helpful in enabling a more complete picture to be established of the biology of the survey area.

The data delivered by such a study can provide evidence for the presence of aerial DNA, from which species presence may be inferred.

In addition to the previously mentioned objectives the following processes regarding `eDNA ecology' may usefully be taken into consideration for eDNA collection and sampling design.

### a) eDNA origin

Attempting to predict the origins of the genetic material collected as eDNA, or to tie its detection to either a species physiological functions (such as metabolism and body mass), particular ecological traits (such as, for example reproduction timing, behaviour, locomotion type, activity patterns, and trophic levels (predators, detritivores) or physical living environment.

### b) eDNA fate

Linking biotic and abiotic environmental conditions contribute to organism detection by eDNA taking in consideration their impact in eDNA persistence, degradation rates and detection capabilities of certain taxonomic groups.

### c) eDNA transport

Relating organism detection by aerial eDNA with the ability of genetic material to be transported through diffusion, mixing, settling, and biological mechanisms and anthropogenic interaction between natural species and humans. For example, analysis can include probability maps that take into consideration prevailing wind directions, as the wind is potentially the strongest mechanism for DNA transport, dispersion and dilution over large distances. Also, biological mechanisms and anthropogenic interactions, (for instance, a bird consuming a worm would release the worm's DNA into the air or a run-over hedgehog (roadkill) may release significant DNA into the air over a prolonged period of time) can be taken into consideration when analysis eDNA analysis results.

Although it is not yet clear it is believed humidity may also affect aerosolization of DNA particles, viruses and cells and thus may influence dispersion. Furthermore, it is believed that for certain collection methods the humidity influences the potential for the aerial DNA to adhere to surfaces.

Weather conditions may also restrict or improve DNA transfer into the air. Weather can influence certain species' behaviour and interactions. Rain and wind may agitate soils and vegetation thereby releasing more DNA left on the particles and surfaces into the air. Furthermore, wet roads will cause more spray mobilizing more DNA from road surfaces. At the same time the rain may result in removal of DNA from collection surfaces through run off or the use of windscreen wipers.

From our trials it is also clear that DNA from marine organisms can be collected through this new approach to material collection even at some distance from the sea, but not during all conditions or always at similar distances from sea. It can be postulated that wave action causes transfer of marine or aquatic DNA into the air. Lastly, it is believed that snow cover or ice may limit DNA transfer from soils, vegetation, or aquatic environments.

Depending on the objective of the study, various weather conditions can be utilised or avoided to enhance recovery of DNA from specific species or taxonomic groups.

### Metadata Collection

For effective processing or interpretation of the data resultant from this invention various metadata are preferably collected alongside the sample itself. These allow comparative conclusions to be drawn between different samples or to provide quality control measures (i.e. preservation applied correctly). Metadata can include transect coordinates, time and date, weather collection, collection surface and other relevant variables.

Most in field cleaning is not completely successful in removing DNA from surfaces or other parts of collection devices. It may therefore be useful to collect data before the device is cleaned and consider these relevant way points when interpreting data subsequently obtained using the cleaned device or surface.

The collection of location coordinates can be done by various commercially available data loggers and even location emitting GPS (or other satellite-based location determining) systems. In addition, for some types of vehicle (e.g. trains, public buses, delivery vehicles, etc.) location data may be captured and held remotely so that the vehicle's location may be tracked from a remote location, and the relevant data, if accessible, may be useful in relating captured biological material to a vehicle's route and timetable.

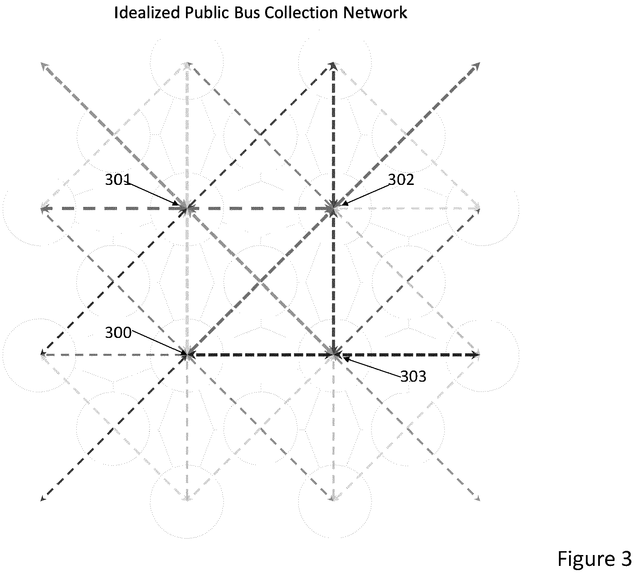

Figure 3 is a map illustrating schematically an idealized transport network utilised for collection of biological samples. In this example the transport network is a bus network. In the example illustrated there are four public transport hubs 300, 301, 302, and 303. Here we assume that the hubs are equally spaced, and each hub is connected by a direct transport route with each other hub. Ideally buses would be traveling back and forth along the same transect day in day out. By sampling DNA from all routes going to each hub a good coverage is achieved.

Furthermore, the area around the central hubs is sampled at least 8 times allowing one to confirm likely presence around the hub or infer likely absence around the hub, thereby making it more likely the DNA originates somewhere along the route away from the hub. The collected biodiversity data from the bus routes and hubs can also be crosschecked via GBIF data overlay, given it retains information's of species occurrence rates and geographic distribution. Further away from the hub there are diagonal routes that also intersect providing another datapoint to confirm presence or infer absence making it more likely DNA originates away from this intersection point. In this way the diagonal routes between hubs could be subdivided into areas in which DNA providence can be inferred and the horizontal and vertical routes can be subdivided into areas in which DNA providence can be inferred (see grey outlined areas).

An additional cost optimization step could be achieved by sampling longer routes (i.e., routes that go through 2 hubs, thick dotted lines) only in one single hub. Using the same principles of intersection explained above this could allow horizontal and vertical routes can be subdivided into 4 areas and diagonal routes to be subdivided into 6 areas.

Also, by sampling multiple times (i.e., duplicate sampling or during multiple days, weeks or months that cover different seasonal species cycles) more accurate results can be achieved or uncertainty can be reduced. Note that for the areas outside the grey circles (depicting intersecting routes or hubs) and along a single route DNA presence/absence cannot be resolved as there is no intersecting route that can be sampled confirming DNA presence or absence. For these areas data like wind velocity, known species presence and ecosystems maps could be used together with machine learning algorithms to add more granularity.

### Public transport Logistical constraints

Based on our interaction with various bus companies, it is evident that cleaning schedules vary. Some buses and trains operating in Western European countries, and countries such as Japan and South Korea, are cleaned every day. This cleaning cannot be expected to remove all collected eDNA on surfaces, but it will have a reducing effect on the amount of biological material remaining on the surface. It is therefore helpful to understand and record the cleaning schedules, but regular cleaning cannot be relied upon in the context of defining a clear collection window (that is, it cannot be assumed that any biological material on a collection surface arrived in the interval since the last cleaning event).

Bus companies may also adopt varying bus routing strategies. It has become apparent that some companies keep the same buses on the same transect, without diverting any of the buses to a different transect (a transect may equate to a route, but a numbered bus route may actually describe different transects on different days or at different times of days - and this of course needs to be taken into account). The same bus company, or other bus companies, may run the same buses on the same transect for extended periods (e.g. for several days or hours, going back and forth along the same transect - although the same transect run in opposite directions may be given differing route identifiers. Such a regime is helpful for our purposes because it gives flexibility about the timing of sample collection, as well as potentially simplifying data processing - as opposed to a situation in which a given bus may take different possible routes (transects) according to demand for and availability of buses.

Other companies routinely redirect buses to new routes based on daily schedules; typically changing the route once the bus reaches the central hub (i.e. a central train station or other public transport hubs). It may be possible to review a bus's schedule one or several days in advance and one could thus plan the collection (and/or cleaning) strategy beforehand. However, in many cases rerouting happens in real time at the bus depot, junction or terminus based on bus availability (for example to use an available bus to replace a failed bus from another route) and it may thus be challenging (if not impossible) to plan cleaning and collection ahead of time. Usually, it is possible to gain insights in the routes that individual buses have travelled either by accessing a centralized platform or via loggers in the buses. In those cases, the strategy may be that buses on selected bus-lines reaching the central hub are sampled, whilst also recording previously travelled routes and using computational processing to allow for the fact the biological material could also have been collected during those previous routes.

It will be appreciated that the use of removable sampling surfaces (e.g., glass or metal panels or sheets, or plastic films or sheets, as described elsewhere), that can be mounted to a vehicle just prior to a journey of the vehicle and then removed from the vehicle after completion of that journey, is particularly helpful in such situations. Such an approach is also very helpful when the vehicle being used for material collection is not a public transport vehicle but instead a vehicle which may have a less circumscribed route - such as a road haulage vehicle or delivery vehicle (whose route details for journeys taken may be acquired during or after the event using the vehicle's navigation system or an on-board tracker).

Collection from public buses can generally occur safely around most public transport hubs or route termini, as only 2 or 3 minutes are typically required to swab the front of the bus using paint rollers and similar times should be achievable with arrangements in which one or more removable collection surfaces are used (and which hence need to be swapped out or simply removed at the end of a transect journey or series of journeys) and other collection devices. Typically, buses stop at least 5 minutes at these central hubs and termini, sometimes longer if bus drivers are changed. Sampling at these public transport hubs is ideal as it allows for daytime collection and more flexibility of selecting routes and times for sampling. Alternatively, sampling can occur at the overnight depots (where most buses are also cleaned), but here sampling time is limited to one moment per day (upon arrival at the overnight depot, although sometimes buses may return to the overnight depot at least once during the day and before the end of the day) and there might not be solid control on the transect travelled by bus companies that utilize a mixed routing strategy. The use of dedicated tracking devices to track individual vehicle's actual journeys is helpful in this respect, particularly if used in conjunction with a vehicle identifier such as a device-readable code (e.g. barcode, QR code, or an RFID or NFC data tag) that is secured to the vehicle in such a location that it can easily be read (for example using a smart phone or other handheld device) by the person collecting the biological material. Of course, a vehicle may be identified using a vehicle registration number on its number plate, but that may require the taking of a photograph or some other record which may require more manual intervention.

When biological samples are collected (harvested from the vehicle) they are preferably each identified by means of a barcode or the like, for example by being placed (e.g. sealed) into sterile containers to which barcodes are already attached. The person doing the harvesting follows an appropriate protocol to ensure that given harvested samples are associated with an identified vehicle and hence an identified transect or series of transects (the records of which will also include dates and times).

### Experimental results

A series of trials were conducted over several months using municipal buses of a small European coastal town. 4 bus routes were chosen, each having different characteristics. The first, A, was a purely urban route (about 10km in length) which neither ran adjacent the sea nor adjacent parkland. The route was essentially linear, with buses turning at each end of the route to make the return journey. The second route, B, was also an urban route, essentially circular, that for several hundred metres the route runs along the sea front, and is also adjacent park land at various points. The third and fourth routes were extra-urban routes. Route C is a linear route that starts and ends in urban areas but which for about half its length (about 10km overall) traverses green space. The fourth route, D, is partially urban but runs for about 2/3 of its length (about 20km overall) alongside the sea, and for a considerable length adjacent parkland. The 4 bus routes all have daily services, and the journeys are in each case generally made with vehicles dedicated to the relevant route. The 4 routes cross zones with different levels of naturalisation and compositions of habitats.

Biological samples were harvested from buses on the 4 routes, typically by swabbing the buses using paint rollers soaked in a phosphate buffer. Samples were collected on average every 4 days, with two samples taken from each bus each time, for a total of 5 harvesting events. DNA was extracted and amplified using 3 primers, resulting in a total of 120 DNA samples. The results were filtered to remove arthropod species. Below we display a table summarizing the DNA samples analysed for each primer as well as the number of non-arthropod species detected in each group.

| Primer | DNA samples | Average counts per sample | Number of features | Non-arthropod species detected |
|---|---|---|---|---|
| 16S gene target for insects | 40 | 55,429 | 6847 | 76 |
| COI general gene target | 40 | 78,873 | 3886 | 19 |
| 16S gene target for mammals | 40 | 68,346 | 2791 | 169 |

There was an overlap of 9 species between the two 16S primers, but no overlap with the species identified using the COI primer. DNA samples were sequenced by high-throughput sequencing, and a computational pipeline was used to assess the presence of known species, by matching the sequenced data to genomics databases. Among all DNA samples, a total of 255 species were observed in at least one biological sample.

| Class | Number of species | Top 10 species |
|---|---|---|
| Aves (Birds, Vulture) | 61 | Gallus gallus; Columba livia; Turdus merula; Streptopelia decaocto; Anas platyrhynchos; Cairina moschata; Curruca melanocephala; |
| | | Melopsittacus undulatus; Columba palumbus; Sylvia atricapilla |
| Actinopteri | 55 | Dicentrarchus labrax; Sardina pilchardus; Salmo salar; Diplodus sargus; Sparus aurata; Sarpa salpa; Dicentrarchus punctatus; Lipophrys pholis; Lipophrys trigloides; Parablennius pilicomis |
| Mammalia (Mammals) | 24 | Bos taurus; Canis lupus; Homo sapiens; Sus scrofa; Rattus norvegicus; Rattus rattus; Oryctolagus cuniculus; Mus spretus; Felis catus; Apodemus sylvaticus |
| Gastropoda (Gastropods, slugs) | 22 | Limacus flavus; Rumina decollata; Rissoa parva; Vallonia costata; Lauria cylindracea; Deroceras laeve; Arion lusitanicus; Alvania tenera; Skeneopsis planorbis; Ferussacia folliculus |
| Clitellata (Clitellata) | 12 | Octodrilus complanatus; Aporrectodea trapezoides; Microscolex dubius; Microscolex phosphoreus; Enchytraeus dichaetus; Eisenia fetida; Octolasion cyaneum; Amynthas corticis/Amynthas diffringens; Enchytraeus japonensis; |
| Bivalvia (Bivalves, Clam) | 11 | Hiatella arctica; Ruditapes philippinarum; Venerupis corrugata; Mytilaster minimus; Striarca lactea; Cerastoderma edule; Abra alba; Donax vittatus; Scrobicularia plana, Abra sp. |
| Amphibia (Amphibians) | 10 | Bufo bufoBufo spinosus; Pelophylax perezi; Lissotriton boscai; Triturus pygmaeus; Pleurodeles waltl; Salamandra salamandra; Discoglossus galganoi; Epidalea calamita; **Pelobates cultripes;** Bufo bufo |
| Gymnolaemata Gymnolaemates, Marine Bryozoans) | 10 | Electra pilosa; Bugulina turbinata; Cryptosula pallasiana; Aetea anguina; Conopeum reticulum; Membranipora membranacea; Scruparia chelata; Amathia sp.; Microporella ordo; Scruparia sp. |
| (unassigned) | 7 | Plasmopara viticola; Parvamoeba rugata; Phaeocystis globosa; Peronospora rifoliorum; Albugo sp.; Peronospora affinis; Peronospora radii |
| Lepidosauria (Lepidosaurs | 5 | Tarentola mauritanica; Podarcis virescens; Anguis fragilis; Blanus cinereus/Blanus mariae; Chalcides chalcides/Chalcides striatus |
| Ophiuroidea (Basket Stars, Brittle stars) | 5 | Amphipholis squamata; Ophiactis balli; Ophiocomina nigra; Ophiura albida; Ophioderma appressa |
| Chondrichthyes (Cartilaginous Fishes) | 4 | Raja microocellata; Prionace glauca; Raja brachyura; *Raja undulata |
| Cephalopoda (Octopuses, Cephalopods) | 3 | Octopus vulgaris; Doryteuthis opalescens; Dosidicus gigas |
| Echinoidea (Sea Urchins, Heart Urchins) | 3 | Psammechinus miliaris; Echinocardium cordatum; Paracentrotus lividus |
| Sordariomycetes Pyrenomycete) | 3 | Lecanicillium saksenae; Diaporthe longicolla; Memnoniella echinata |
| Asteroidea (Sea Stars, starfish) | 2 | Marthasterias glacialis; Asterina gibbosa |
| Demospongiae (Demosponges) | 2 | Mycale contarenii; Tetilla sp. |
| Enopla | 2 | Emplectonema sp.; Prostoma sp. |
| Eurotatoria | 2 | Adineta vaga; Philodina citrina |
| Eurotiomycetes | 2 | Penicillium brevicompactum; Aspergillus puulaauensis |
| Florideophyceae | 2 | Symphyocladiella dendroidea; Apoglossum ruscifolium |
| Holothuroidea (Sea cucumbers) | 2 | Holothuria forskali; Holothuria mammata |
| Hydrozoa (Hydroids, Hydralike Animals) | 2 | Liriope tetraphylla; Obelia dichotoma |
| Dothideomycetes | 1 | Cladosporium cladosporioides |
| Palaeonemertea | 1 | Cephalothrix rufifrons |
| Polychaeta (Bristle Worms, Paddle-Footed Annelids) | 1 | Chaetopterus variopedatus |
| Thaliacea | 1 | Doliolum nationalis |

It is notable that so many marine species were detected in these samples taken from the fronts of buses that had merely driven within proximity to the sea (within about 50 metres for bus route D). Interestingly, however, 19 of the 55 fish species (actinopteri) were detected in samples from at least 3 of the 4 bus routes. One of the four identified species of chondrichthyes, raja undulata is classed as "endangered" and the others are all classed as "near threatened". And on one of the bus routes DNA from two "near threatened" species of parrot was detected.

The same approach was used to identify insect species using the 120 DNA samples harvested from the fronts of the buses, again using the COI and two 16S primers.

| Primers | DNA samples | Average counts per sample | Number of features | Insect species detected |
|---|---|---|---|---|
| 16S gene target for insects | 40 | 55,429 | 6847 | 410 |
| COI general gene target | 40 | 78,873 | 3886 | 293 |
| 16S gene target for mammals | 40 | 68,346 | 2791 | 124 |

DNA samples were sequenced by high-throughput sequencing, and a computational pipeline was used to assess the presence of known species, by matching the sequenced data to genomics databases. Among all DNA samples, a total of 657 species were observed in at least one biological sample.

There was an overlap of 105 species between the two 16S primers, 30 of which were also detected using the COI primer. A further 7 species were detected using both 16S gene target for mammals and the COI primers. A further 28 species were identified using both the 16S gene target for insects and COI primers. 277 species were identified only using the 16S gene target for insects, 228 were only identified using the COI primer and 12 further species were only identified using the 16S gene target for mammals. It can therefore be seen that it is sensible to use multiple primers in order to increase the number of species identified. If monitoring for plant rather than animal DNA suitable primers should of course be used, for example ITS2.

### DNA Extraction and Amplification

Total DNA was extracted from the samples collected from the fronts of the buses according to an internally optimized procedure designed to maximize the recovery of insect DNA. The DNA samples underwent quality control before PCR amplification and library preparation. All samples meeting the established quality control thresholds were further processed.

Two molecular markers representing insect populations were analysed for each sample. A single Illumina sequencing library was prepared for each DNA sample and marker using an in-house-optimized protocol.

Laboratory controls, negative (molecular grade pure water) and positive (pure insect DNA), were added to the analysis and processed in parallel with the DNA samples. All laboratory controls yielded the expected results. All sequencing libraries underwent multiple quality control steps at each stage of the processing and proceeded to sequencing.

### DNA Sequencing

Libraries were sequenced on Illumina MiSeq in paired-end 300 mode, resulting in 2 X 13,015,461 reads.

The sequencing run performance was within the platform specifications. Only high-quality sequencing reads were used for bioinformatic analysis.

### Metabarcode Analysis

Sequenced reads were analysed using a custom metabarcode analysis pipeline based on the qiime2 platform. Briefly, quality control of raw sequencing data was performed in order to verify sequencing accuracy. Reads not containing the amplification primer sequences were discarded. The remaining were used to construct a set of representative sequences (ASVs) and filtered to discard amplicons of unexpected length, resulting in 6847, 3886 and 2791 sequences for 16S gene target for insects, COI and 16S gene target for mammals respectively. A set of classifiers were then trained in order to assign these sequences to taxonomic groups resulting in a total of 2132 high confidence taxa. Figures and additional analyses were made using custom R and Python scripts.

### Quality Control

All results were evaluated and classified according to internally established quality control thresholds and only high confidence species calls were reported.

The positive controls were validated through the identification of the insect species used in the analysis. No species were detected in negative control samples.

### Databases

The following databases were accessed in the process of this analysis: MIDORI version GB245.

### Resolving spatial and temporal resolution

Our methods will be able to sample eDNA over long transects and at different time-points and convert those data to detected species. To further refine the spatial and temporal resolution of the detections, we use data analysis and combine different transects. The following paragraphs describe one such possible approach.

As the eDNA is collected over a transect and over a period of time the location and time of the moving collection surface could be used to map the potential areas from which the eDNA originates (potential occurrence map), together with an associated probability score. Further data like weather conditions, time of day, (alterations in) travel velocity, collection surface type, landscape features would also be integrated to increase the temporal and spatial resolution or to reduce uncertainty in occurrence data. Furthermore, data from other transects or that are collected during another time, could be used to refine the potential occurrence map, by combining the probabilities originating from each transect. Lastly, data from other sources (manual surveys, occurrence data bases, etc.) may be integrated to refine the potential occurrence map. The potential occurrence map may be linked to a specific period or point in time allowing the temporal resolution to be captured. The inference of the potential occurrence map can include machine learning techniques or rule-based heuristics. The final result would be a geographically and temporally indexed dataset describing the probability of detection of specific taxa.

A potential approach would involve a machine-learning classification algorithms such as neural networks or random forests. One model for each species would be created. Inputs for these models would be the metadata associated with the samples (location, time of day, landscape features, data in other databases associated with the same location, etc.) and the target for training would be whether we detected the model's species in our samples. After training, the output of the model would be a confidence of probability that the species could be detected in this time and location. By using a suitably trained classifier we could extend this to allow us to predict species presence even without sampling, provided the metadata exists. For example, we could train classifiers that link the eDNA that we have detected to various other (spatial, etc.) data (i.e. forest cover, temperature, existence of other species) and in that way allows us to predict the likelihood of detecting the eDNA in an unsampled area for which such other data exist.

Another approach could be based on the known route of the collection vehicle. The unity detection of a species in the whole transect would be divided into a uniform probability distribution over the length of the transect. Finally, the information from several transects would be combined by adding the probabilities of presence on intersecting routes. This approach could be done for all samples or by sub-setting for variables such as date, time of say or meteorological conditions.

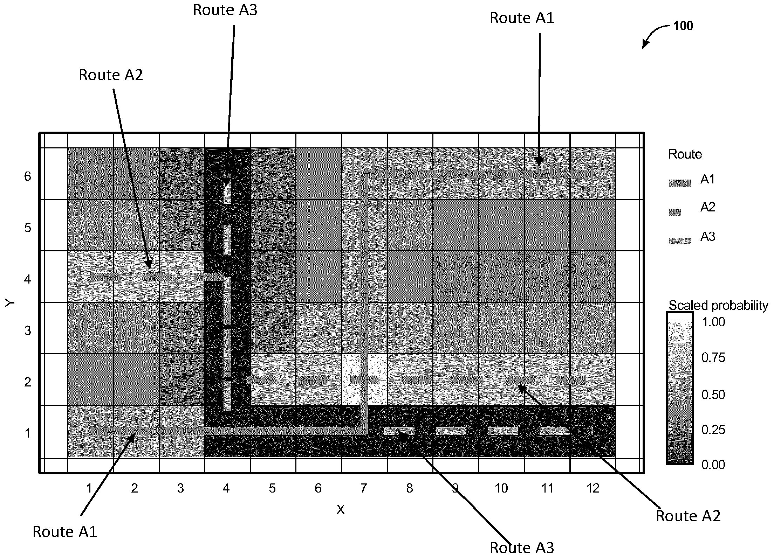

Figure 4 illustrates a possible approach to the generation and use of probability maps. To generate the probability maps that reflect the probability from where the eDNA was collected within a transect transected survey area, the survey area is divided into grid cells of a to be defined size (based on the required resolution, the overall size of the survey area, and the density of transects within the area). For example, if we consider the whole of the Netherlands, we could achieve a 10km by 10km resolution using approximately 360 bus lines strategically selected over the entire country; a 5km by 5km resolution could be achieved if we also added some other collection types - such as delivery trucks. For a municipal area with reasonable bus line density (or reasonable density of some other public transport routes) we could realistically expect to achieve a resolution down to 2km by 2km, or even 1km by 1km. The probability of collection (0<p<1) is determined per grid cell based on mathematical calculations and comparing multiple routes and sample events.

Figure 4 that shows a survey zone or area (here the area is shown as a rectangle of 6 units by 12 units) that includes three vehicle routes (A1, A2 & A3) which may for example be public transport routes (e.g., bus routes). For the purpose of discussion, we assume that each of these routes is sampled on a weekly basis, with the sampling events for the three routes all taking place on the same day, although these conditions/restrictions are clearly not essential. For example, the routes could be sampled daily or every few days, and the different routes could be sampled with different frequencies and at different times.

Routes A1 and A3 overlap from cell (4,1) to cell (7,1); route A1 crosses over route A2 in cell (7,2); and route A2 overlaps with route A3 from cell (4,2) to cell (4,4).

On route A1 a species S1 is identified at T0 (first sampling event). This means that somewhere along the transect defined by the route eDNA of species S1 has been collected (assuming the sampling surface was fully clean at the start of the sampling period). The transect for route A1 crosses a certain number of grid cells (g_{A1}), in this case 17. Without knowing more, we have to assume an equal probability along the transect of the route. For each grid cell through which route A1 runs the `probability of collection' p_{S1,A1}=1/g_{A1} is thus here equal to (1/17=0.059). The algorithm then moves on to incorporate the next route A2, whose transect crosses 14 cells. Species S1 was also identified on this route and hence the probability for each grid cell is p_{S1},_{A2} = 1/g_{A2} (1/14=0.071). On route A3 species S1 was not detected, hence p_{S1},_{A3} = f for all grid cells through which the transect for this route runs. Here f is a factor that is adjusted to reflect a chance of a false negative. In this example we assume PSI,A3 = 0.

In cells through which a route does not pass a large scale (at the size of the average route length) moving average is applied using the probability of collection of all routes sampled. This moving average is applied for each `probability of collection' grid linked to each route. It is based on other `probability of collection' grid cells through which a route does pass. The moving average algorithm could also be another exploration algorithm like Kriging (Gaussian process regression) or multipoint statistics, and or using secondary data (such as vegetation cover, land use maps, wind velocities, other species occurrence databases) for guidance. In our example we use one average value for all cells that are further than one grid cell distance from a route, and for all cells within one grid cell distance a "neighbouring" average being the average of all such neighbouring cells (being cells near a route). For this we first calculate a base probability for cells distant from (all) routes based on the mean probabilities of the routes in the (total) survey area. For this example this would be p_{S1,A1} = (p_{S1,A1} + p_{S1},_{A2} + p_{S1},_{A3})/3 = (0.059 + 0.071 + 0)/3=0.043. At a larger scale, in which some routes are shorter than the total survey area, a moving average would be applied.

The aggregated/modelled probability of collection for species S1 (psi) for each grid cell is P_{S1,A1} × p_{S1},_{A2} × p_{S1},_{A3}. This means the lack of detection of species S1 on route A3 sets the probability of collection for the cells in which other routes (A1 & A2) are intersecting that route also to 0 in our example. If two or more routes in which a given species was detected intersect in a cell, the probability of collection for that species in that cell is increased (as shown, for example, for species S1 in cell 7,2, where routes A1 and A2 intersect).

For instance, grid cell (7,2) would have a probability of 0.059 × 0.071 × 0.043=0.00018. In grid cells (4,1);(5,1);(6,1) & (7,1) this would be 0.059 × 0.071 × 0 = 0 (again assuming there is no chance of false negatives; f=0). In other non-intersecting grid cells along route A1 the probability is 0.059 × 0.043 × 0.043=0.00011, for route A2 that is 0.043 × 0.071 × 0.043=0.00014. For all other cells (outside the routes) the probability initially is 0.043 × 0.043 × 0.043=0.00008. After, for those grid cells next to, but not part of, a route, we calculate the mean of the probabilities of (in this case) the 3x3 square around it, to reflect the added information that we have about the surrounding area. We may apply smoothing based on a larger scale (for example based on the use of a 9x9 square, or larger). The grading of the cells in Figure 4 reflects these values.

As the aggregated/modelled probability map of collection is not a true reflection of the likelihood linked to species presence its values can be scaled or normalized. Also, in subsequent sampling moments (T1, T2, etc) the probability for grid cells could be recalculated and added to the previous probability map. Data from many timesteps back could be used with lesser weight to also generate smooth temporal trends and maps. Alternatively, the data from previous timesteps could be used as guide for the moving average or Kriging algorithm.

### Example:

One example of this concept is shown in our test using buses in 2 provinces (A and B) of in the Netherlands. Here we detected `microtus arvalis' (the common vole) from all 4 sampled bus lines in province A. In province B we only detected it from one of the 3 lines we sampled and very consistently in that single line (5 consecutive buses traveling the same line). In the other 2 lines the common vole was not detected. This corresponds well with known occurrences (reported via citizen science) at a high resolution (5x5km grid cells) as the common vole is reported in and around the city in which we sampled the buses in province A. In province B there is more of a pattern and the common vole was not reported in the grid cells transected by the two lines in which it was not detected, nor near the central hub we sampled. Instead, the common vole is mainly reported at the end of the specific line in which we found it consistently.

Also, the `myodes glareolus' (bank vole) was found in none of the buses sampled in province A, while it was detected in 4 out of 7 buses sampled in province B. This corresponds closely with the known occurrence of myodes glareolus which is hardly reported in province A.

In most urban areas the local mass transit network already provides a good basis for sampling. We have sampled in four bus routes in the municipality of about 100km2, representing less than 10% of the total number of routes in the area. The full set covers a larger area and has many intersecting routes as well as routes that overlap for sections with multiple stops in common.

### Data Delivery and Presentation

For some end users the delivery and presentation of the rich data this delivers may require special data platform, websites, software or dashboards (interactive graphic platforms for data explorations). In such software applications the species information derived from the `Bio-Informatics processing' and/or `Resolving of the spatial and temporal resolution' computation steps is integrated and presented as graphs, maps, metrics, lists and interactive menus. These data presentations can include summary metrics and indexes focusing on ecological aspects of the client's interest, including, but not limited to:
- life-stages evaluation - determination of whether the detected species are in larval or adult stage, for example;
- function/metabolic potential - by detecting bacterial taxa and/or their gene-coding sequences, we will be able to determine the which metabolic processes the bacterial community present in our samples can do;
- ecosystem services - ecologists have assigned services to a variety of species. These can range from the biological (like pollination) to the environmental (soil degradation) to the cultural (species of cultural importance like butterflies)
- functional networks mapping - identifying the biological interactions (predator-prey, parasite, symbiosis) between species detected in the same location using existing databases such as GloBI (Global Biotic Interactions);
- pollination capacity - by identifying the detected arthropod species that are known to pollinate the plant species known to occur in a location;
- soil health- by identifying among the detected bacterial and fungal species those known in the literature to represent healthy and unhealthy agricultural soil;
- threatened species and invasive species monitoring - by identifying among the detected species those present in endangered and invasive species databases, such as that of the IUCN;
- general biodiversity and population trends.

The adding of another layer of outsourced information (i.e. remote sensing, satellite data, artificial intelligence, ecological modelling) to the DNA-based observations would allow the detection of multi-systemic ecological changes and for creation of prediction models.

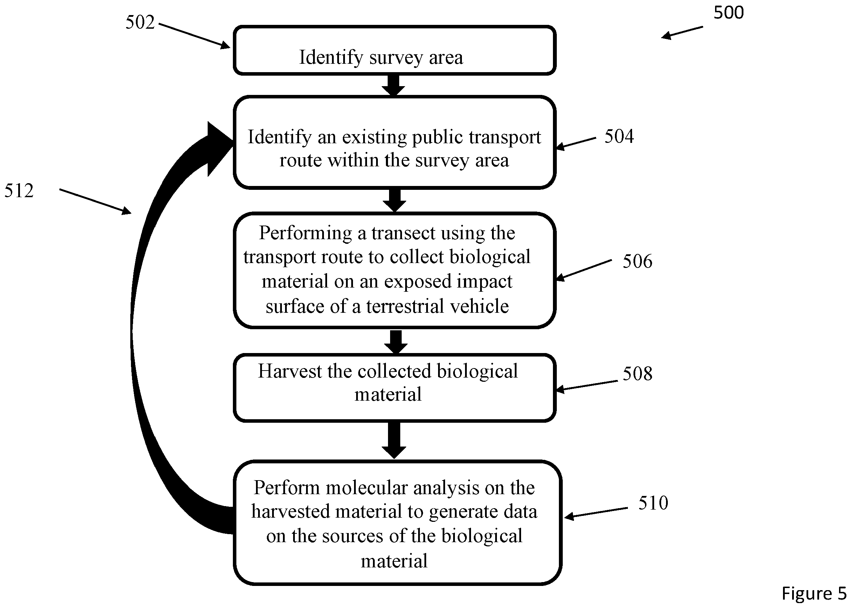

Figure 5 is a flow chart to illustrate a method 500 according to an aspect of the invention. As a first step 502 a geographical survey area is identified. This may for example be as the result of a request from a client (an individual, a business, a local, regional, or national governmental organisation, an international organisation, a quango, etc.) or the result of a desire to determine some aspect of biological or ecological presence within the survey area.

In this example of the method, it is decided to take advantage of existing public transport routes as a means to collect material samples, so at step 504 one or more existing public transport routes are identified (e.g. selected) for initial acquisition of samples. At step 506, transects of the survey area are performed using the or each selected public transport route - which may for example be just a single transport type (e.g., bus, tram or train), or a mix of different transport types e.g. bus and tram, or bus and train, or bus tram, and train. Collection may be performed over periods of hours within a single day or 24 hour period, or over several days, or even longer, as described earlier. For each route a single vehicle may be used, or multiple vehicles, depending upon timetabling and logistics.

After a predetermined collection period (which may differ for some or more of the routes, or which may be standardised for the different routes), the collected biological material for each route is harvested at step 508.

Following harvesting, at step 510 molecular analysis is performed as previously described.

The process may be continued 512 based on the identification/selection of further existing public transport routes. Optionally, the further public transport routes may be selected based on the results of the molecular analysis - for example based on the further public transport routes intersecting the routes initially chosen. It will be appreciated that the method illustrated in Figure 5 could equally well make use of road haulage vehicles driven on known (or capturable) road haulage routes through the survey area, or some combination of public transport vehicles and road haulage vehicles. Equally, private hire vehicles, such as school buses and/or tourist buses might be used as well as or instead of public transport vehicles, and references to public transport vehicles should be considered to include such vehicles unless the context clearly requires otherwise. In some cases, it may be desirable only to use public transport vehicles and not private vehicles.

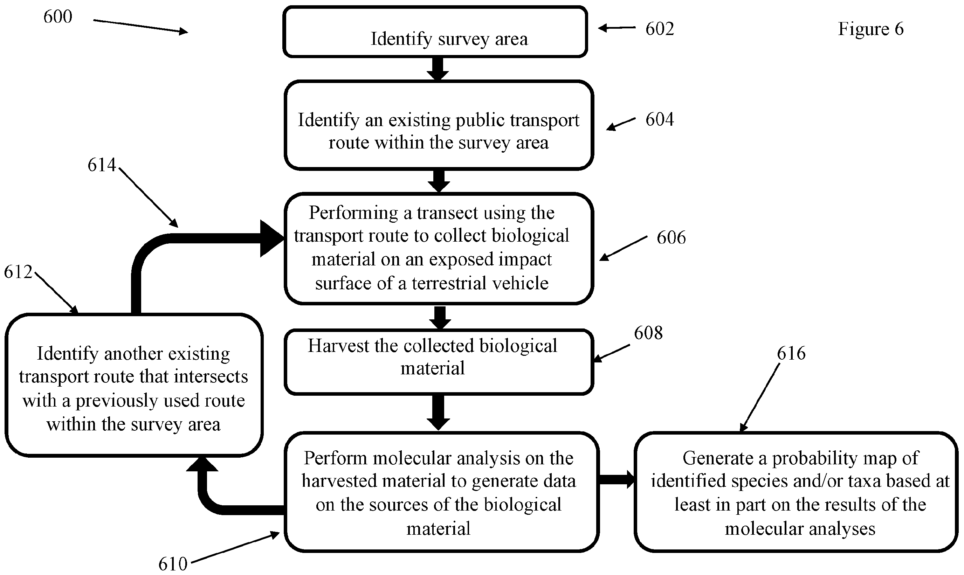

Figure 6 illustrates a method 600 that corresponds generally to the method 500 of Figure 5, with initial survey area identification, 602, identification 604 of an existing public transport route, collection of biological material at 606, harvesting at 608, and molecular analysis at step 610. But in this case the results of molecular analysis at step 610 are used as basis for selection 612 of one or more additional public transport routes that intersect with a previously used route within the survey area. The collection, harvesting and analysis steps 606-610 are then repeated. There may be further iterations of step 612 in order to acquire a fuller picture of biological presence within the survey area, and optionally extending beyond the initial survey area. At step 616 a probability map of identified species and/or taxa may then be generated based at least in part on the results of the molecular analyses performed at step 610.

### 1.1 EXAMPLE 1 - ECOLOGICAL METRICS

### 1.1.1 EXAMPLE - BIODIVERSITY REPORTING REQUIREMENTS

A business may need to provide metrics around biodiversity impact in relation to its assets. This is linked to regulatory reporting requirements in various countries, including the EU. Such a company will have to list biodiversity metrics in and around the site, more specifically presence of endangered species. The approach to biological harvesting and the provision of biodiversity metrics described in this patent application may be applied by harvesting biological material from vehicles traversing transects around the site but beyond its perimeter, as well as from vehicles traversing transects on the site itself. Depending upon the scale and geography of the site this may require the use of drones or other aerial vehicles. It may be possible to perform at least some of the external (beyond the site perimeter) transects using public transport. Indeed, eDNA results determined based on material gathered during "routine" surveys using for example public transport vehicles could be used as the basis of the off-site biodiversity metrics. Businesses with large sites and/or those having reason to be concerned about local biodiversity may choose to work together with local public transport services (and/or other operators of vehicles traversing the relevant zones) to perform routine and regular (at least monthly, for example) monitoring based on vehicle-harvested biological samples. A data platform that is based on the data collected using a technique according to an aspect of the invention could also provide this information through `data as a service'.

The ever greater onus on businesses to take responsibility for the impact on biodiversity means that there should be a ready market for reliable data on biodiversity, such as that acquired using a method according to an aspect of the present invention. By conducting sufficiently frequent geographically extensive and thorough reviews of biodiversity based on methods according to aspects of this invention it would be possible to establish a reliable database of the biodiversity "status quo"- tailored if necessary for locally endangered or rare species. Such a database could provide the foundation for a site-specific biodiversity report that could be "topped up" with on-site surveys using the vehicle-based biological harvesting technique according to aspects of the invention.

Such a database is likely to be of particular value in areas containing industrial plant and sites, and of great interest to local or regional councils/government.

### 1.1.2 EXAMPLE - ENVIRONMENTAL IMPACT ASSESSMENT

For the construction of a large infrastructure project an environmental assessment has to be made. Ecological consultants typically conduct a desktop screening prior to visiting the site. The desktop screening could be based on a database established as just described based on biological samples collected using a method according to an aspect of this invention.

### 1.1.3 EXAMPLE - ENVIRONMENTAL MONITORING OF GOVERNMENTAL POLICY

For policies at regional level, like limitations on pesticides, reduced nitrogen run-off from agricultural land, restrictions in the use or discharge of toxic substances, etc. the ecological impact may have to be monitored to showcase the positive impact (for example in terms of increased biodiversity or the maintenance of endangered species) to various stakeholder. A data platform of the type previously described could also usefully the basis for such comparative examination of biodiversity and biodiversity trends.

### 1.1.4 EXAMPLE - REGIONAL BIODIVERSITY TREND DATA

Various countries have to report on national or regional biodiversity trends either as part of international agreements or due to their own laws and obligations. The collection of trend data on a large number of species and sampled in a consistent way, achieved through this invention, can provide this information to governments as well as potentially supporting NGOs and international bodies in their monitoring roles.

### 1.1.5 EXAMPLE - SCIENTIFIC STUDY DATA

Scientific ecological studies often rely on other reports or (public) data sources to i) design the study or survey and ii) draw the final conclusions. By making the biodiversity data acquired using methods according to aspects of the invention available to researchers, NGOs, scientific institutes, or other non-profit organisation they can gain a stronger understanding of ecological networks, derive empirical relationships, advance science and better understand biodiversity changes and impacts.

### 1.2 EXAMPLE 2 - INVASIVE, ALIEN, HARMFUL & ILLEGAL SPECIES

### 1.2.1 EXAMPLE - INVASIVE SPECIES MONITORING FOR GOVERNMENTS

Invasive species are one of the key threats to biodiversity and for that reason many governments have policies in place to monitor, locate and control or eradicate these invasive species. The lists of such species are constantly evolving as new information becomes available and known invasive species still spread into new areas. By using the vehicle-based harvesting techniques described in the context of this invention it becomes possible to carry out what are in effect routine large scale (geographically extensive and frequent) watches for such species, providing basis to determine trends and to spot new occurrences at relatively low cost. Moreover, in the event that a new species is added to the list of alien of harmful species it is possible for biological material previously harvested using methods according to aspects of the invention to be re-examined to check for presence of the new species.

### 1.2.2 EXAMPLE - CROP PLAGUE TRENDS DETECTION FOR FARMERS

There are many known plant pests that occur in and around farmland. Currently such pests are often eradicated through preventative use of pesticides which causes damage to other species and may not be necessary at all or applied at a high dose due to lack of detailed information on the threat. In various countries pesticide usage is being lowered through regulation as it is very damaging to biodiversity and also to human health. With spatial and temporal data regarding the presence of pest (largely invertebrate, but occasionally also vertebrate) and plague (generally microbial) provided by methods according to aspects of the invention, farmers can better develop pesticide strategies (i.e., Integrated Pest Management; IPM) and tailor their usage allowing minimal damage while ensuring food security.

### 1.2.3 EXAMPLE - QUARANTINE SPECIES DETECTION FOR GOVERNMENTS

Apart from commonly occurring pests there are also various species listed as quarantine species. These species are not prevalent in an area and if they were to be introduced, they could seriously damage crops. For that reason, governments maintain lists of quarantine species that are to be checked upon import of goods (phytosanitary control) or have regulation regarding quarantine times for plant-based material. Regardless of these checks the quarantine species still make their way into new areas and if left unchecked are very difficult to eradicate. Aspects of the present invention provide effective and low cost techniques to support an early monitoring network that would allow actions to be taken more rapidly, potentially reducing the risk of significant financial damage to the agricultural sector.

### 1.2.4 EXAMPLE - DAMAGING INSECT OR FUNGAL MONITORING FOR INSURANCE COMPANIES

In various countries insects like termites, woodboring beetles and/or fungi (e.g. dry rot and moulds) can cause serious damage to buildings or result in health issues to the inhabitants. Here often management and eradication programs are in place and the areas with high risk are known. However, with the spread of the species through globalization and climate change it is likely that new regions will be affected. Here structural damage may be severe due to lack of eradication and management methods (i.e. certain wood preservation techniques, ventilation measures or frequent checks). For property owners or insurance companies seeking to manage the risks caused from this damage aspects of the present invention can be used to provide spatial and temporal data regarding these pests that may allow their prompt management, with consequent reduction in damage and loss.

### 1.2.5 EXAMPLE - DETECTION OF ILLEGALLY KEPT PETS OR ILLEGAL CONSUMPTION FOR AUTHORITIES

In many countries the trade, possession, or consumption of protected or endangered species is illegal. The collecting techniques of aspects of the invention, described in the context of eDNA, can also be applied to the policing of the relevant regulations. As mentioned in the context of the experiments performed with biological material collected across 4 bus routes, DNA from two near threatened" species of parrot was detected - demonstrating the applicability of the basic idea. By applying methods according to aspects of the invention to intersecting transects performed frequently it should be possible to narrow down the area for investigation to reveal the location of the illicit act(s).

### 1.2.6 EXAMPLE - ANIMAL DISEASE (VECTOR, VIRAL, BACTERIAL, FUNGAL) MONITORING

Partly due to intensive animal farming there has been a rise in cases of large outbreaks of viral, bacterial, or fungal diseases. In some cases, the spread is clearly airborne, in other cases it occurs through direct contact or via vectors (mostly insects, but also other species). Methods according to aspects of the invention can be used to support regional monitoring covering insects, bacteria, fungi, and viruses so that hotspots and spreading patterns may be identified. This can help animal welfare & health authorities and farmers to better manage such risk and control further spread.

### 1.3 EXAMPLE 3 - HEALTH MONITORING

### 1.3.1 EXAMPLE - HUMAN DISEASE VECTOR MONITORING FOR HEALTH AUTHORITIES

A prime example of a human disease vector is the mosquito that spreads Malaria and Dengue. The tiger mosquito is of especial concern to northern countries as this vector seems to be spreading, partly due to climate change. In some countries eradication and control measures are in place but practically these need to be focused, and currently such focusing relies on communal sightings or other localized surveys, seriously limiting the chance of detection. The methods according to aspects of the invention could provide an improved alternative to current sighting and survey techniques, offering a greater chance of containment and potentially eradication. Applying the methods according to aspects of the invention to regional monitoring covering insects, hotspots and spreading patterns of such human/animal disease vectors can be identified allowing surveys and eradication to be more focussed.

### 1.3.2 EXAMPLE - VIRAL EPIDEMIOLOGY FOR HEALTH AUTHORITIES

The recent COVID-19 epidemic has stressed the importance of early viral outbreak detection. During COVID many governments adopted a wastewater surveillance system that preceded detection of many of the clinical cases. Potentially by also monitoring airborne viral presence using the methods outlined in this patent a more extensive regional monitoring method can be deployed. For certain viruses such a method could even provide an earlier metric of infection rates, prevalence and/or infection risk with a larger spatial coverage. Adopting an untargeted detection and screening for viruses not yet present in an area could also provide a very early detection method potentially allowing mitigation of further spread. The WHO, Governments and Health authorities could benefit significantly from such data. A likely constraint here is the viability of viruses and viral particles in the air and once they have attached to the impact surface. Depending upon the nature of the impact surface, UV light levels, and temperature, there may be only a relatively brief window in which to harvest and process samples in order to be able to reveal the presence a virus. But if these factors are taken into account, and sampling and processing are done expeditiously it may be possible to use the techniques described in this patent application in monitoring for viral presence.

### 1.3.3 EXAMPLE - PATHOGEN PREVALENCE FOR INSURERS

It is known that in certain regions or areas of cities there are higher occurrences of certain illnesses. In some cases, this has demographic causality, but it may also be due to other environmental factors or the presence of pathogens. One of the oldest examples was the analysis performed by John Snow in 1854 which revealed that an outbreak of cholera was linked to one specific water pump. More recently, Legionella has been linked to public fountains, but only after deducting a commonly visited location by all people falling ill. Through the methods outlined in this patent health authorities can take more focused actions to prevent new outbreaks or more easily understand and determine sources of clinical cases.

## Claims

1. A method of generating information about the biology within a survey zone, the method comprising:
i) harvesting biological material from an external impact surface that has been carried through the survey zone by a terrestrial vehicle, the surface having been exposed to air that is displaced by movement of the vehicle through the survey zone;
ii) performing molecular analysis on the biological material to generate data on sources of the biological material.

2. The method of claim 1, wherein the harvesting involves swabbing the external impact surface to transfer biological material from the external impact surface to an article used to swab the external impact surface, wherein optionally the swabbing is performed using a paint roller.

3. The method of claim 1, wherein the harvesting involves washing the external impact surface to transfer biological material from the surface to a liquid used to wash the surface.

4. The method of any one of the preceding claims, wherein the external impact surface is provided by a body part of the vehicle.

5. The method of any one of claims 1 to 3, wherein the external impact surface is part of a collecting element carried on the exterior of the vehicle.

6. The method of claim 5, wherein the collecting element is a flexible film or sheet, and optionally wherein the flexible film or sheet is adhered to an external surface of the vehicle.

7. The method of claim 5, wherein the collecting element comprises a glass or metal panel.

8. A method of generating information on biological presence within a geographical survey area, the method comprising:
i) collecting biological material on an external impact surface of a terrestrial vehicle moved on one or more first transects through the survey area;
ii) harvesting the collected biological material from the impact surface;
iii) performing molecular analysis on the harvested biological material to generate data on sources of the biological material.

9. A method as claimed in claim 8, further comprising creating a database based on the results of the molecular analysis performed in step iii).

10. A method as claimed in claim 9, further comprising populating the database with biological presence data from multiple iterations of the method.

11. A method as claimed in claim 10, further comprising populating the database with biological presence data derived from iterations of the method performed for different geographical areas.

12. A method as claimed in any one of claims 8 to 11, wherein the geographical area is a construction, commercial or industrial site, and the method further comprises collecting biological material on an external impact surface of a vehicle moved over one or more geographical zones beyond a periphery of the site, the harvesting and molecular analysis steps also being performed on biological material so collected.

13. A method as claimed in any one of claims 8 to 11, the method further comprising the steps of:
iv) performing one or more further captures of biological material on one or more second transects through the survey area, each of the one or more second transects intersecting with one or more of the one or more first transects;
v) harvesting the collected biological material from the impact surface;
vi) performing molecular analysis on the harvested biological material to generate data on sources of the biological material;
vii) generating a probability map of identified species and/or taxa based at least in part on the results of the molecular analyses performed in steps iv) and vi).

14. A method as claimed in claim 13, wherein at least one of the one or more second transects is selected based at least in part on results of the analysis performed in step iii).

15. A method of monitoring a geographical area for the presence of a DNA, RNA, or protein based marker indicative of the presence or functioning of a biological target, the method comprising:
i) collecting biological material on an external impact surface of a terrestrial vehicle moved over a geographic area;
ii) harvesting the collected biological material from the impact surface;
iii) performing molecular analysis on the harvested biological material to generate data indicative of the presence or absence or functioning of the biological target.

16. A method as claimed in claim 15, wherein the biological target is a rare, threatened or endangered species.

17. A method as claimed in claim 15, wherein the biological target is a species targeted for control or eradication, and optionally wherein the biological target is a termite, woodboring beetle, or fungi such as dry rot.

18. The method of any one of the preceding claims, wherein the terrestrial vehicle is a public transport vehicle such as a bus, tram, or train.

19. A method as claimed in any one of the preceding claims, wherein the method is performed using more than one vehicle type, and optionally wherein the method is performed both using a terrestrial collection vehicle and an aerial collection vehicle.
